# EUROPEAN PATENT SPECIFICATION

(11) **EP 4 147 052 B1**
(45) Date of publication and mention of the grant of the patent: **28.10.2026**
(21) Application number: 21722251.2
(22) Date of filing: 04.05.2021
(51) Int. Cl.: A61K 39/00, C12Q 1/6886, G01N 33/5752, G01N 33/575

(54) **PREDICTING RESPONSE TO PD-1 AXIS INHIBITORS**
VORHERSAGE DER REAKTION AUF PD-1-ACHSEN-INHIBITOREN
PRÉDICTION DE RÉPONSE À DES INHIBITEURS DE L'AXE PD-1

(30) Priority: 05.05.2020 EP 20172842
(43) Date of publication of application: 15.03.2023
(73) Proprietor: F. Hoffmann-La Roche AG, 4070 Basel (CH)
(72) Inventor: GASSER, Stephan, 8952 Schlieren (CH); ROLLER, Andreas, 4070 Basel (CH)
(74) Representative: Klostermeyer-Rauber, Dörte
(86) International application number: PCT/EP2021/061638
(87) International publication number: WO 2021/224215

(56) References cited:
- WO-A1-2021/030156
- US-A1- 2018 094 322
- KLEMPNER SAMUEL J. ET AL: "Tumor Mutational Burden as a Predictive Biomarker for Response to Immune Checkpoint Inhibitors: A Review of Current Evidence", THE ONCOLOGIST, vol. 25, no. 1, 1 January 2020 (2020-01-01), XP055817575, ISSN: 1083-7159, Retrieved from the Internet <URL:https://www.ncbi.nlm.nih.gov/pmc/articles/PMC6964127/pdf/ONCO-25-e147.pdf> DOI: 10.1634/theoncologist.2019-0244
- SHEN JIANFENG ET AL: "ARID1A deficiency promotes mutability and potentiates therapeutic antitumor immunity unleashed by immune checkpoint blockade", NATURE MEDICINE, NATURE PUB. CO, NEW YORK, vol. 24, no. 5, 7 May 2018 (2018-05-07), pages 556 - 562, XP036513459, ISSN: 1078-8956, [retrieved on 20180507], DOI: 10.1038/S41591-018-0012-Z
- ROY S. HERBST ET AL: "Predictive correlates of response to the anti-PD-L1 antibody MPDL3280A in cancer patients", NATURE, vol. 515, no. 7528, 26 November 2014 (2014-11-26), London, pages 563 - 567, XP055262130, ISSN: 0028-0836, DOI: 10.1038/nature14011
- LU?S FELIPE CAMPESATO ET AL: "Comprehensive cancer-gene panels can be used to estimate mutational load and predict clinical benefit to PD-1 blockade in clinical practice", ONCOTARGET, vol. 6, no. 33, 1 October 2015 (2015-10-01), United States, pages 34221 - 24227, XP055415163, DOI: 10.18632/oncotarget.5950
- LUIS FELIPE CAMPESATO: "Comprehensive cancer-gene panels can be used to estimate mutational load and predict clinical benefit to PD-1 blockade in clinical practice - Supplementary Material", 1 October 2015 (2015-10-01), XP055611165, Retrieved from the Internet <URL:https://www.oncotarget.com/article/5950/text/> [retrieved on 20190806], DOI: 10.18632/oncotarget.5950
- XU ZHENGLIN ET AL: "Overexpression of the ASPM gene is associated with aggressiveness and poor outcome in bladder cancer", ONCOLOGY LETTERS, vol. 17, 1 January 2019 (2019-01-01), GR, pages 1865 - 1876, XP055818096, ISSN: 1792-1074, Retrieved from the Internet <URL:https://www.ncbi.nlm.nih.gov/pmc/articles/PMC6341836/pdf/ol-17-02-1865.pdf> DOI: 10.3892/ol.2018.9762

## Description

### Field of the Invention

The present invention relates to biomarkers for predicting response of a patient with a cancer to a PD-1 axis inhibitor such as an anti-PD-L1 antibody or an anti-PD1 antibody. Provided herein is a method of identifying a cancer patient responsive to a PD-1 axis inhibitor by determining presence of gene transcript implicated in the maintainance of stem cells in a tumor tissue sample. Specifically, the invention pertains to an in vitro method of identifying a patient having cancer who is responsive to a therapy comprising an effective amount of an anti-PD-L1 antibody, the method comprising determining the abundance of stem cell maintenance-related genes in a tumor tissue sample obtained from a patient having cancer, wherein the abundance of stem cell maintenance-related genes is characterized by detecting the expression level of all genes selected from a group consisting of ASPM, CNOT3, LRP5 and PBX1 and wherein the method further comprises a step of comparing the expression level of the genes to a reference level, whereby an increased expression level is indicative of response to a therapy comprising an effective amount of an anti-PD-L1 antibody.

### Background of the Invention

PD-1 is an immunoglobulin superfamily member discovered on 1992 as a gene upregulated in T cell hybridoma undergoing cell death (Ishida et al., 1992, EMBO J, 11: 3887-95). PD-1 is mainly found on activated T, B and myeloid cells. The important negative regulatory function of PD-1 was revealed by autoimmune-prone phenotype of Pdcd1-/- mice in 1999 (Nishimura et al., 1999, Immunity, 11: 141-51). In 1999 PD-L1 (B7-H1), the first ligand of PD-1, was identified (Dong et atl., 1999, Nat Med, 5: 1365-9), followed by PD-L2 (B7-DC) in 2001 (Latchman et al., 2001, Nat Immunol, 2: 261-8). Another costimulatory molecule, the CD80 (B7-1) interacts specifically with PD-L1 (Butte et al., 2007, Immunity, 27: 111-22) as well. PD-1 contains two immunoreceptor tyrosine-based motifs that are phosphorylated upon receptor engagement and recruit Src homology 2-domain-containing tyrosine phosphatase 2. The PD-1:PD-L1 pathway inhibits T cell proliferation by reducing the production of IL-2 and restricts the number of T cells that gain entry into the cell cycle as well as their subsequent division rate. Up-regulation of PD-L1 expression was described in several human tumors types, which hijacks the PD-L1 to interact with PD-1 on T cells and suppress effector function. These findings led to the successful clinical application of PD-1 blockade in treating solid tumors (Sharma et al., 2015, Cell, 161: 205-14). Nevertheless, so far only a minor subset of patients (<30%) benefit from such a therapy, with as-yet unknown mechanisms (Zou et al., 2016, Sci Transl Med, 8: 328rv4). Roy S. Herbst et al.: "Predictive correlates of response to the anti-PD-L1 antibody MPDL3280A in cancer patients",NATURE, vol. 515, no. 7528, 26 November 2014 (2014-11-26), pages 563-567, discloses genes that are upregulated during response to the anti-PD-L1 antibody MPDL3280A in cancer patients.

Accordingly, there is a need for methods for determing which patients are likely to benefit from a therapy with a PD-1 axis inhibitor such as an anti-PD-L1 antibody that inhibits the binding of PD-L1 to PD-1.

The potential of self-renewal and differentiation from the cell of origin is referred to as stemness (Miranda et al., 2019, PNAS 116 (18), 9020-9029). Deregulation of gene expression during tumorgenesis can often lead to gain of a stem cell-like phenotype and the loss of properties associated with differentiation. Acquisition of stem cell-related phenotypes correlates with increased metastasis potential of tumor cells (Friedmann-Morvinski and Verma, 2014, EMBO Reports 15(3), 244-53; Ge et al., 2017; Shibueand Weinberg, 2017; Visvader and Lindeman, 2012; Bradneret al., 2017; Young, 2011). Recent evidence suggests that stem cells and cancer stem cells also have immune modulatory properties and stem cell-like phenotypes of tumors were associated with the presence of tumor infiltrating lymphocytes. In accordance, immune pressure has been shown to select or induce tumors with a stem cell-like phenotype.

Recent studies have provided evidence that the stem cell-like phenotype can be described by basic gene-expression programs in various cancer types (Malta et al., 2018, Cell 173(2), 338-354). Building on this prior work, we destilled a core set of genes from the stem cell maintenance gene ontology term list whose expression at base line inversely correlates with overall survival in patients treated with Atezolizumab. Using public single-cell RNA sequencing data from melanoma and HNSCC patients, the core set of stem cell-related genes negatively correlated with differentiation antigens and genes implicated in antigen presentation. Loss of antigen presentation and differentiation antigens have previously been implicated in immune escape (McGranahan et al., 2017, Cell 171(6), 1259-1271).

### Summary of the Invention

Hereinafter, the terminology "embodiment" relates to the disclosure and only forms part of the invention if it falls within the scope of the claims.

Provided herein is evidence that the analysis of tumor biopsy at baseline from patients with renal cell carcinoma who received treatment with an anti-PD-L1 antibody, atezolizumab, and showed that patients with lower expression of genes related to stem cell-like phenotypes had a significant survival advantage as compared to those with lower expressions. Thus, the data support the notion that a stem cell-like phenotype is linked to pathways that enable immune escape or immune suppression. The absence of stem cell-like features in tumors is predictor of a better clinical outcome in response to a therapy with a PD-1 axis inhibitor such as PD-L1 blockade treatment.

The present invention relates to an in vitro method of identifying a patient having cancer who is responsive to a therapy comprising an effective amount of an anti-PD-L1 antibody, the method comprising determing the abundance of stem cell maintenance-related genes in a tumor tissue sample obtained from a patient having cancer.

The abundance of stem cell maintenance-related genes is characterized by detecting the expression level of ASPM, CNOT3, LRP5 and PBX1. In one particular aspect, the abundance of stem cell maintenance-related genes is characterized by detecting the expression level a group consisting of ASPM, CNOT3, LRP5 and PBX1.

The gene signatures of the invention and the methods of detecting the expression of genes within the gene signatures allow the identification and determination of those individuals afflicted with cancer, tumors, or neoplasms who may, or who are likely to, respond to treatment with a PD-1 axis inhibitor.

In one aspect, the method thus further comprises a step of comparing the expression level of the one or more genes to a reference level, whereby an increased expression level is indicative of the response to a therapy comprising an effective amount of a PD-1 axis inhibitor. In one particular aspect, increased expression level indicates that the individual afflicted with cancer, tumors, or neoplasms is less likely to respond to treatment with a PD-1 axis inhibitor.

In one aspect, the expression level is detected in the sample by protein expression. In another aspect, the expression level is detected in the sample by mRNA expression.

In a further aspect, the expression level is detected using a method selected from the group consisting of FACS, Western blot, ELISA, immunoprecipitation, immunohistochemistry, immunofluorescence, radioimmunoassay, immunodetection methods, mass spectrometery, HPLC, qPCR, RT-qPCR, multiplex qPCR or RT-qPCR, RNA-seq, microarray analysis, nanostring, SAGE, MassARRAY technique, and FISH, and combinations thereof.

In another aspect, provided is an *in vitro* method of identifying a patient having cancer who is responsive to a therapy comprising an effective amount of a PD-1 axis inhibitor as described herein before, wherein the cancer is selected from the group consisting of non-small cell lung cancer, small cell lung cancer, renal cell cancer, colorectal cancer, ovarian cancer, breast cancer, pancreatic cancer, gastric carcinoma, bladder cancer, esophageal cancer, mesothelioma, melanoma, head and neck cancer, thyroid cancer, sarcoma, prostate cancer, glioblastoma, cervical cancer, thymic carcinoma, leukemia, lymphomas, myelomas, mycoses fungoids, merkel cell cancer, and other hematologic malignancies. In one particular aspect, the cancer is locally advanced or metastatic non-small cell lung cancer or urothelial bladder cancer. In one particular aspect, the cancer is locally advanced or metastatic non-small cell lung cancer.

In one aspect, provided is an *in vitro* method of identifying a patient having cancer who is responsive to a therapy comprising an effective amount of a PD-1 axis inhibitor as described herein before, wherein the therapy comprises an effective amount of a PD-1 axis inhibitor as monotherapy.

In one further aspect, provided is an *in vitro* method of identifying a patient having cancer who is responsive to a therapy comprising an effective amount of a PD-1 axis inhibitor as described herein before, wherein the therapy comprises an effective amount of a PD-1 axis inhibitor and an effective amount of a second agent selected from the group consisting of a cytotoxic agent, a chemotherapeutic agent, a growth inhibitory agent, a radiation therapy agent, and anti-angiogenic agent, and combinations thereof.

In one aspect, the PD-1 axis inhibitor is a PD-1 binding antagonist. In one further aspect, the PD-1 binding antagonist inhibits the binding of PD-1 to PD-L1. In one aspect, the PD-1 binding antagonist is an anti-PD-1 antibody. In another aspect, the PD-1 axis inhibitor is a PD-L1 binding antagonist. In one further aspect, the PD-L1 binding antagonist inhibits the binding of PD-L1 to PD-1. In one aspect, the PD-L1 binding antagonist is an anti-PD-L1 antibody. In one further aspect, the anti-PD-L1 antibody is an antibody fragment selected from the group consisting of Fab, Fab'-SH, Fv, scFv, and (Fab')₂. In a further aspect, the anti-PD-L1 antibody is selected from the group consisting of atezolizumab, avelumab, durvalumab and MDX-1105. In one particular aspect, the anti-PD-L1 antibody is atezolizumab.

In one further aspect, provided is an *in vitro* method of identifying a patient having cancer who is responsive to a therapy comprising an effective amount of a PD-1 axis inhibitor as described herein before, wherein the tumor tissue sample is a sample obtained from the patient prior to the therapy with a PD-1 axis inhibitor.

Disclosed but not claimed is a pharmaceutical composition comprising a PD-1 axis inhibitor for use in the treatment of a patient having cancer, wherein the patient is determined to be responsive to a therapy comprising an effective amount of a PD-1 axis inhibitor in accordance with the method as described herein before.

Disclosed but not claimed is a method of determining whether a patient having cancer is more suitably to be treated by a therapy comprising an effective amount of a PD-1 axis inhibitor, the method comprising determing the abundance of stem cell maintenance-related genes in a tumor tissue sample obtained from a patient having cancer.

Disclosed but not claimed is a method of improving the treatment effect of a therapy comprising an effective amount of a PD-1 axis inhibitor in a patient having cancer, the method comprising determing the abundance of stem cell maintenance-related genes in a tumor tissue sample obtained from a patient having cancer.

Disclosed but not claimed is a method of treating a patient having cancer, comprising the steps of determining the abundance of stem cell maintenance-related genes in a tumor tissue sample obtained from a patient having cancer, predicting if the patient is is responsive to a therapy comprising an effective amount of a PD-1 axis inhibitor, and administering to the patient a therapy comprising an effective amount of a PD-1 axis inhibitor.

Disclosed but not claimed is a method of treating a patient having locally advanced or metastatic non-small cell lung cancer, comprising the steps of determining the abundance of stem cell maintenance-related genes in a tumor tissue sample obtained from a patient having cancer, predicting if the patient is is responsive to a therapy comprising an effective amount of a PD-1 axis inhibitor, and administering to the patient a therapy comprising an effective amount of a PD-1 axis inhibitor

Disclosed but not claimed is a method of treating a patient having urothelial bladder cancer, comprising the steps of determining the abundance of stem cell maintenance-related genes in a tumor tissue sample obtained from a patient having cancer, predicting if the patient is is responsive to a therapy comprising an effective amount of a PD-1 axis inhibitor, and administering to the patient a therapy comprising an effective amount of a PD-1 axis inhibitor.
These and other aspects are further described in the detailed description below.

### Brief Description of the Figures

**Figures 1A** **and** **1B** show the Kaplan-Meier survival curves in patients with locally advanced or metastatic Non-Small Cell Lung Cancer (OAK). Overall Survival (%) curves are shown in Figure 1A, whereas Figure 1B shows progression-free survival (%). Expression of genes related to the abundance of stem cell maintenance-related cancer cells anti-correlates with the survival advantages by a PD-1 axis inhibitor atezolizumab.
**Figure 2** shows the correlation of the stem cell maintenance-related cancer cells genes among each other.
**Figures 3A** **and** **3B** show the Kaplan-Meier survival curves in patients with locally advanced or metastatic Non-Small Cell Lung Cancer (BIRCH). Expression of genes related to the abundance of stem cell maintenance-related cancer cells anti-correlates with the survival advantages by a PD-1 axis inhibitor atezolizumab. Overall Survival (%) curves are shown in Figure 3A, whereas Figure 3B shows progression-free survival (%).
**Figure 4** shows the correlation of the stem cell maintenance-related cancer cells genes among each other
**Figures 5A** **and** **5B** show the Kaplan-Meier survival curves in patients with locally advanced or metastatic Urothelial Bladder Cancer (IMvigor211). Overall Survival (%) curves are shown in Figure 5A, whereas Figure 5B shows progression-free survival (%).Expression of genes related to the abundance of stem cell maintenance-related cancer cells anti-correlates with the survival advantages by a PD-1 axis inhibitor atezolizumab.
**Figure 6** shows the correlation of the stem cell maintenance-related cancer cells genes among each other.

### Detailed Description

### Definitions

The term "PD-1 axis inhibitor" is a molecule that inhibits the interaction of a PD-1 axis binding partner with either one or more of its binding partner, so as to remove T-cell dysfunction resulting from signaling on the PD- 1 signaling axis - with a result being to restore or enhance T-cell function, e.g., proliferation, cytokine production, target cell killing. As used herein, a PD- 1 axis inhibitor includes a PD- 1 binding antagonist and a PD-L1 binding antagonist.

The term "PD-1 binding antagonist" is a molecule that decreases, blocks, inhibits, abrogates or interferes with signal transduction resulting from the interaction of PD-1 with one or more of its binding partners, such as PD-L1, PD-L2. In some aspects, the PD-1 binding antagonist is a molecule that inhibits the binding of PD-1 to its binding partners. In a specific aspect, the PD-1 binding antagonist inhibits the binding of PD- 1 to PD-L1 and/or PD-L2. For example, PD-1 binding antagonists include anti-PD-1 antibodies, antigen binding fragments thereof, immunoadhesins, fusion proteins, oligopeptides and other molecules that decrease, block, inhibit, abrogate or interfere with signal transduction resulting from the interaction of PD- 1 with PD-L1 and/or PD-L2. In one aspect, a PD-1 binding antagonist reduces the negative co-stimulatory signal mediated by or through cell surface proteins expressed on T lymphocytes mediated signaling through PD-1 so as render a dysfunctional T-cell less dysfunctional, e.g. enhancing effector responses to antigen recognition. In some aspects, the PD-1 binding antagonist is an anti-PD-1 antibody. In one specific aspect, a PD-1 binding antagonist is nivolumab (MDX- 1106). In another specific aspect, a PD-1 binding antagonist is pembrolizumab (Merck 3745). In one further specific aspect, a PD-1 binding antagonist is cemiplimab (REGN-2810). In one specific aspect, a PD-1 binding antagonist is spartalizumab (PDR001). In one further specific aspect, a PD-1 binding antagonist is camrelizumab (SHR1210). In one specific aspect, a PD-1 binding antagonist is sintilimab (IBI308). In another specific aspect, a PD-1 binding antagonist is PD1-0103 or humanized versions thereof as described in WO 2017/055443 A1.

The term "PD-L1 binding antagonist" is a molecule that decreases, blocks, inhibits, abrogates or interferes with signal transduction resulting from the interaction of PD-L1 with either one or more of its binding partners, such as PD-1, B7-1. In some aspects, a PD-L1 binding antagonist is a molecule that inhibits the binding of PD-L1 to its binding partners. In a specific aspect, the PD-L1 binding antagonist inhibits binding of PD-L1 to PD-1 and/or B7-1. In some aspects, the PD-L1 binding antagonists include anti-PD-L1 antibodies, antigen binding fragments thereof, immunoadhesins, fusion proteins, oligopeptides and other molecules that decrease, block, inhibit, abrogate or interfere with signal transduction resulting from the interaction of PD-L1 with one or more of its binding partners, such as PD-1 and B7-1. In one aspect, a PD-L1 binding antagonist reduces the negative co-stimulatory signal mediated by or through cell surface proteins expressed on T lymphocytes mediated signaling through PD- L1 so as to render a dysfunctional T-cell less dysfunctional, e.g. enhancing effector responses to antigen recognition. In some aspects, a PD-L1 binding antagonist is an anti-PD-L1 antibody. In one specific aspect, an anti-PD-L1 antibody is atezolizumab. In another specific aspect, an anti-PD-L1 antibody is avelumab. In still another specific aspect, an anti-PD-L1 antibody is durvalumab. In yet another specific aspect, an anti-PD-L1 antibody is MDX-1105.

The term "antibody" herein is used in the broadest sense and encompasses various antibody structures, including but not limited to monoclonal antibodies, polyclonal antibodies, multispecific antibodies, e.g., bispecific antibodies, and antibody fragments so long as they exhibit the desired antigen-binding activity.

The terms "anti-PD-L1 antibody" and "an antibody that binds to PD-L1" refer to an antibody that is capable of binding PD-L1 with sufficient affinity such that the antibody is useful as a diagnostic and/or therapeutic agent in targeting PD-L1. In one embodiment, the extent of binding of an anti- PD-L1 antibody to an unrelated, non-PD-L1 protein is less than about 10% of the binding of the antibody to PD-L1 as measured, e.g., by a radioimmunoassay (RIA). In certain embodiments, an anti-PD-L1 antibody binds to an epitope of PD-L1 that is conserved among PD-L1 from different species.

A "blocking" antibody or an "antagonist" antibody is one which inhibits or reduces biological activity of the antigen it binds. Preferred blocking antibodies or antagonist antibodies substantially or completely inhibit the biological activity of the antigen.

An "antibody fragment" refers to a molecule other than an intact antibody that comprises a portion of an intact antibody that binds the antigen to which the intact antibody binds. Examples of antibody fragments include but are not limited to Fv, Fab, Fab', Fab'-SH, F(ab')₂; diabodies; linear antibodies; single-chain antibody molecules (e.g., scFv); and multispecific antibodies formed from antibody fragments.

An "antibody that binds to the same epitope" as a reference antibody refers to an antibody that blocks binding of the reference antibody to its antigen in a competition assay by 50% or more, and conversely, the reference antibody blocks binding of the antibody to its antigen in a competition assay by 50% or more.

The term "chimeric" antibody refers to an antibody in which a portion of the heavy and/or light chain is derived from a particular source or species, while the remainder of the heavy and/or light chain is derived from a different source or species.

The terms "full length antibody," "intact antibody," and "whole antibody" are used herein interchangeably to refer to an antibody having a structure substantially similar to a native antibody structure or having heavy chains that contain an Fc region as defined herein.

The term "monoclonal antibody" as used herein refers to an antibody obtained from a population of substantially homogeneous antibodies, i.e., the individual antibodies comprising the population are identical and/or bind the same epitope, except for possible variant antibodies, e.g., containing naturally occurring mutations or arising during production of a monoclonal antibody preparation, such variants generally being present in minor amounts. In contrast to polyclonal antibody preparations, which typically include different antibodies directed against different determinants (epitopes), each monoclonal antibody of a monoclonal antibody preparation is directed against a single determinant on an antigen. Thus, the modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by a variety of techniques, including but not limited to the hybridoma method, recombinant DNA methods, phage-display methods, and methods utilizing transgenic animals containing all or part of the human immunoglobulin loci, such methods and other exemplary methods for making monoclonal antibodies being described herein.

A "human antibody" is one which possesses an amino acid sequence which corresponds to that of an antibody produced by a human or a human cell or derived from a non-human source that utilizes human antibody repertoires or other human antibody-encoding sequences. This definition of a human antibody specifically excludes a humanized antibody comprising non-human antigen-binding residues.

A "humanized" antibody refers to a chimeric antibody comprising amino acid residues from non-human CDRs and amino acid residues from human FRs. In certain aspects, a humanized antibody will comprise substantially all of at least one, and typically two, variable domains, in which all or substantially all of the CDRs (e.g., HVRs) correspond to those of a non-human antibody, and all or substantially all of the framework regions (FRs) correspond to those of a human antibody. A humanized antibody optionally may comprise at least a portion of an antibody constant region derived from a human antibody. A "humanized form" of an antibody, e.g., a non-human antibody, refers to an antibody that has undergone humanization.

The term "detection" includes any means of detecting, including direct and indirect detection.

As used herein, the term "reagent that specifically detects expression levels" refers to reagents used to detect the expression of one or more genes (e.g., including but not limited to, the cancer markers of the present invention). Examples of suitable reagents include but are not limited to, nucleic acid probes capable of specifically hybridizing to the gene of interest, aptamers, PCR primers capable of specifically amplifying the gene of interest, and antibodies capable of specifically binding to proteins expressed by the gene of interest.

The term "biomarker" as used herein refers to an indicator, e.g., predictive, diagnostic, and/or prognostic, which can be detected in a sample. The biomarker may serve as an indicator of a particular subtype of a disease or disorder (e.g., cancer) characterized by certain, molecular, pathological, histological, and/or clinical features. In some aspects, a biomarker is a gene. Biomarkers include, but are not limited to, polynucleotides (e.g., DNA, and/or RNA), polynucleotide copy number alterations (e.g., DNA copy numbers), polypeptides, polypeptide and polynucleotide modifications (e.g. posttranslational modifications), carbohydrates, and/or glycolipid-based molecular markers.

The terms "biomarker signature," "signature," "biomarker expression signature," or "expression signature" are used interchangeably herein and refer to one or a combination of biomarkers whose expression is an indicator, e.g., predictive, diagnostic, and/or prognostic. The biomarker signature may serve as an indicator of a particular subtype of a disease or disorder (e.g., cancer) characterized by certain molecular, pathological, histological, and/or clinical features. In some aspects, the biomarker signature is a "gene signature." The term "gene signature" is used interchangeably with "gene expression signature" and refers to one or a combination of polynucleotides whose expression is an indicator, e.g., predictive, diagnostic, and/or prognostic. In some embodiments, the biomarker signature is a "protein signature." The term "protein signature" is used interchangeably with "protein expression signature" and refers to one or a combination of polypeptides whose expression is an indicator, e.g., predictive, diagnostic, and/or prognostic.

The "amount" or "level" of a biomarker associated with an increased clinical benefit to an individual is a detectable level in a biological sample. These can be measured by methods known to one skilled in the art and also disclosed herein. The expression level or amount of biomarker assessed can be used to determine the response to the treatment.

The terms "level of expression" or "expression level" in general are used interchangeably and generally refer to the amount of a biomarker in a biological sample. "Expression" generally refers to the process by which information (e.g., gene-encoded and/or epigenetic) is converted into the structures present and operating in the cell. Therefore, as used herein, "expression" may refer to transcription into a polynucleotide, translation into a polypeptide, or even polynucleotide and/or polypeptide modifications (e.g., posttranslational modification of a polypeptide). Fragments of the transcribed polynucleotide, the translated polypeptide, or polynucleotide and/or polypeptide modifications (e.g., posttranslational modification of a polypeptide) shall also be regarded as expressed whether they originate from a transcript generated by alternative splicing or a degraded transcript, or from a post-translational processing of the polypeptide, e.g., by proteolysis. "Expressed genes" include those that are transcribed into a polynucleotide as mRNA and then translated into a polypeptide, and also those that are transcribed into RNA but not translated into a polypeptide (for example, transfer and ribosomal RNAs). Gene expression can be regulated at many stages in the process. "Up-regulation" or "activation" refers to regulation that increases the production of gene expression products (e.g., RNA or protein), while "down-regulation" or "repression" refers to regulation that decrease production. Molecules (e.g., transcription factors) that are involved in up-regulation or down-regulation are often called "activators" and "repressors," respectively.

The term "reference level" herein refers to a predetermined value. As a skilled person will appreciate the reference level is predetermined and set to meet the requirements in terms of e.g. specificity and/or sensitivity. These requirements can vary, e.g. from regulatory body to regulatory body. It may for example be that assay sensitivity or specificity, respectively, has to be set to certain limits, e.g. 80%, 90% or 95%. These requirements may also be defined in terms of positive or negative predictive values. Nonetheless, based on the teaching given in the present invention it will always be possible to arrive at the reference level meeting those requirements. In one embodiment the reference level is determined in healthy individuals. The reference value in one embodiment has been predetermined in the disease entity to which the patient belongs. In certain embodiments the reference level can e.g. be set to any percentage between 25% and 75% of the overall distribution of the values in a disease entity investigated. In other aspects, the reference level can e.g. be set to the median, tertiles or quartiles as determined from the overall distribution of the values in a disease entity investigated. In one aspect, the reference level is set to the median value as determined from the overall distribution of the values in a disease entity investigated.

In certain aspects, the term "increase", "increased" or "above" refers to a level above the reference level.

"Amplification," as used herein generally refers to the process of producing multiple copies of a desired sequence. "Multiple copies" mean at least two copies. A "copy" does not necessarily mean perfect sequence complementarity or identity to the template sequence. For example, copies can include nucleotide analogs such as deoxyinosine, intentional sequence alterations (such as sequence alterations introduced through a primer comprising a sequence that is hybridizable, but not complementary, to the template), and/or sequence errors that occur during amplification.

The term "multiplex-PCR" refers to a single PCR reaction carried out on nucleic acid obtained from a single source (e.g., an individual) using more than one primer set for the purpose of amplifying two or more DNA sequences in a single reaction.

"Stringency" of hybridization reactions is readily determinable by one of ordinary skill in the art, and generally is an empirical calculation dependent upon probe length, washing temperature, and salt concentration. In general, longer probes require higher temperatures for proper annealing, while shorter probes need lower temperatures. Hybridization generally depends on the ability of denatured DNA to reanneal when complementary strands are present in an environment below their melting temperature. The higher the degree of desired homology between the probe and hybridizable sequence, the higher the relative temperature which can be used. As a result, it follows that higher relative temperatures would tend to make the reaction conditions more stringent, while lower temperatures less so. For additional details and explanation of stringency of hybridization reactions, see Ausubel et al., Current Protocols in Molecular Biology, Wiley Interscience Publishers, (1995).

"Stringent conditions" or "high stringency conditions", as defined herein, can be identified by those that: (1) employ low ionic strength and high temperature for washing, for example 0.015 M sodium chloride/0.0015 M sodium citrate/0.1% sodium dodecyl sulfate at 50°C; (2) employ during hybridization a denaturing agent, such as formamide, for example, 50% (v/v) formamide with 0.1% bovine serum albumin/0.1% Ficoll/0.1% polyvinylpyrrolidone/50mM sodium phosphate buffer at pH 6.5 with 750 mM sodium chloride, 75 mM sodium citrate at 42°C; or (3) overnight hybridization in a solution that employs 50% formamide, 5 x SSC (0.75 M NaCl, 0.075 M sodium citrate), 50 mM sodium phosphate (pH 6.8), 0.1% sodium pyrophosphate, 5 x Denhardt's solution, sonicated salmon sperm DNA (50 µg/ml), 0.1% SDS, and 10% dextran sulfate at 42°C, with a 10 minute wash at 42°C in 0.2 x SSC (sodium chloride/sodium citrate) followed by a 10 minute high-stringency wash consisting of 0.1 x SSC containing EDTA at 55°C.

"Moderately stringent conditions" can be identified as described by Sambrook et al., Molecular Cloning: A Laboratory Manual, New York: Cold Spring Harbor Press, 1989, and include the use of washing solution and hybridization conditions (e.g., temperature, ionic strength and %SDS) less stringent that those described above. An example of moderately stringent conditions is overnight incubation at 37°C in a solution comprising: 20% formamide, 5 x SSC (150 mM NaCl, 15 mM trisodium citrate), 50 mM sodium phosphate (pH 7.6), 5 x Denhardt's solution, 10% dextran sulfate, and 20 mg/ml denatured sheared salmon sperm DNA, followed by washing the filters in 1 x SSC at about 37-50°C. The skilled artisan will recognize how to adjust the temperature, ionic strength, etc. as necessary to accommodate factors such as probe length and the like.

The technique of "polymerase chain reaction" or "PCR" as used herein generally refers to a procedure wherein minute amounts of a specific piece of nucleic acid, RNA and/or DNA, are amplified as described in U.S. Pat. No. 4,683,195 issued 28 July 1987. Generally, sequence information from the ends of the region of interest or beyond needs to be available, such that oligonucleotide primers can be designed; these primers will be identical or similar in sequence to opposite strands of the template to be amplified. The 5' terminal nucleotides of the two primers may coincide with the ends of the amplified material. PCR can be used to amplify specific RNA sequences, specific DNA sequences from total genomic DNA, and cDNA transcribed from total cellular RNA, bacteriophage or plasmid sequences, etc. See generally Mullis et al., Cold Spring Harbor Symp. Quant. Biol., 51: 263 (1987); Erlich, ed., PCR Technology, (Stockton Press, NY, 1989). As used herein, PCR is considered to be one, but not the only, example of a nucleic acid polymerase reaction method for amplifying a nucleic acid test sample, comprising the use of a known nucleic acid (DNA or RNA) as a primer and utilizes a nucleic acid polymerase to amplify or generate a specific piece of nucleic acid or to amplify or generate a specific piece of nucleic acid which is complementary to a particular nucleic acid.

"Quantitative real time polymerase chain reaction" or "qRT-PCR" refers to a form of PCR wherein the amount of PCR product is measured at each step in a PCR reaction. This technique has been described in various publications including Cronin et al., Am. J. Pathol. 164(1):35-42 (2004); and Ma et al., Cancer Cell 5:607-616 (2004).

The term "microarray" refers to an ordered arrangement of hybridizable array elements, preferably polynucleotide probes, on a substrate.

The term "polynucleotide," when used in singular or plural, generally refers to any polyribonucleotide or polydeoxyribonucleotide, which may be unmodified RNA or DNA or modified RNA or DNA. Thus, for instance, polynucleotides as defined herein include, without limitation, single- and double-stranded DNA, DNA including single- and double-stranded regions, single- and double-stranded RNA, and RNA including single- and double-stranded regions, hybrid molecules comprising DNA and RNA that may be single-stranded or, more typically, double-stranded or include single- and double-stranded regions. In addition, the term "polynucleotide" as used herein refers to triple- stranded regions comprising RNA or DNA or both RNA and DNA. The strands in such regions may be from the same molecule or from different molecules. The regions may include all of one or more of the molecules, but more typically involve only a region of some of the molecules. One of the molecules of a triple-helical region often is an oligonucleotide. The term "polynucleotide" specifically includes cDNAs. The term includes DNAs (including cDNAs) and RNAs that contain one or more modified bases. Thus, DNAs or RNAs with backbones modified for stability or for other reasons are "polynucleotides" as that term is intended herein. Moreover, DNAs or RNAs comprising unusual bases, such as inosine, or modified bases, such as tritiated bases, are included within the term "polynucleotides" as defined herein. In general, the term "polynucleotide" embraces all chemically, enzymatically and/or metabolically modified forms of unmodified polynucleotides, as well as the chemical forms of DNA and RNA characteristic of viruses and cells, including simple and complex cells.

The term "oligonucleotide" refers to a relatively short polynucleotide, including, without limitation, single-stranded deoxyribonucleotides, single- or double-stranded ribonucleotides, RNA:DNA hybrids and double- stranded DNAs. Oligonucleotides, such as single- stranded DNA probe oligonucleotides, are often synthesized by chemical methods, for example using automated oligonucleotide synthesizers that are commercially available. However, oligonucleotides can be made by a variety of other methods, including *in vitro* recombinant DNA-mediated techniques and by expression of DNAs in cells and organisms.

The term "diagnosis" is used herein to refer to the identification or classification of a molecular or pathological state, disease or condition (e.g., cancer). For example, "diagnosis" may refer to identification of a particular type of cancer. "Diagnosis" may also refer to the classification of a particular subtype of cancer, e.g., by histopathological criteria, or by molecular features (e.g., a subtype characterized by expression of one or a combination of biomarkers (e.g., particular genes or proteins encoded by said genes)).

The term "sample," as used herein, refers to a composition that is obtained or derived from a subject and/or individual of interest that contains a cellular and/or other molecular entity that is to be characterized and/or identified, for example based on physical, biochemical, chemical and/or physiological characteristics. For example, the phrase "disease sample" and variations thereof refers to any sample obtained from a subject of interest that would be expected or is known to contain the cellular and/or molecular entity that is to be characterized. Samples include, but are not limited to, primary or cultured cells or cell lines, cell supernatants, cell lysates, platelets, serum, plasma, vitreous fluid, lymph fluid, synovial fluid, follicular fluid, seminal fluid, amniotic fluid, milk, whole blood, blood-derived cells, urine, cerebro-spinal fluid, saliva, sputum, tears, perspiration, mucus, tumor lysates, and tissue culture medium, tissue extracts such as homogenized tissue, tumor tissue, cellular extracts, and combinations thereof.

By "tissue sample" or "cell sample" is meant a collection of similar cells obtained from a tissue of a subject or individual. The source of the tissue or cell sample may be solid tissue as from a fresh, frozen and/or preserved organ, tissue sample, biopsy, and/or aspirate; blood or any blood constituents such as plasma; bodily fluids such as cerebral spinal fluid, amniotic fluid, peritoneal fluid, or interstitial fluid; cells from any time in gestation or development of the subject. The tissue sample may also be primary or cultured cells or cell lines. Optionally, the tissue or cell sample is obtained from a disease tissue/organ. The tissue sample may contain compounds which are not naturally intermixed with the tissue in nature such as preservatives, anticoagulants, buffers, fixatives, nutrients, antibiotics, or the like.

A "reference sample", "reference cell", "reference tissue", "control sample", "control cell", or "control tissue", as used herein, refers to a sample, cell, tissue, standard, or level that is used for comparison purposes. In one embodiment, a reference sample, reference cell, reference tissue, control sample, control cell, or control tissue is obtained from a healthy and/or non-diseased part of the body (e.g., tissue or cells) of the same subject or individual. For example, healthy and/or non-diseased cells or tissue adjacent to the diseased cells or tissue (e.g., cells or tissue adjacent to a tumor). In another embodiment, a reference sample is obtained from an untreated tissue and/or cell of the body of the same subject or individual. In yet another embodiment, a reference sample, reference cell, reference tissue, control sample, control cell, or control tissue is obtained from a healthy and/or non-diseased part of the body (e.g., tissues or cells) of an individual who is not the subject or individual. In even another embodiment, a reference sample, reference cell, reference tissue, control sample, control cell, or control tissue is obtained from an untreated tissue and/or cell of the body of an individual who is not the subject or individual.

For the purposes herein a "section" of a tissue sample is meant a single part or piece of a tissue sample, e.g. a thin slice of tissue or cells cut from a tissue sample. It is understood that multiple sections of tissue samples may be taken and subjected to analysis, provided that it is understood that the same section of tissue sample may be analyzed at both morphological and molecular levels, or analyzed with respect to both polypeptides and polynucleotides.

By "correlate" or "correlating" is meant comparing, in any way, the performance and/or results of a first analysis or protocol with the performance and/or results of a second analysis or protocol. For example, one may use the results of a first analysis or protocol in carrying out a second protocols and/or one may use the results of a first analysis or protocol to determine whether a second analysis or protocol should be performed. With respect to the aspect of polypeptide analysis or protocol, one may use the results of the polypeptide expression analysis or protocol to determine whether a specific therapeutic regimen should be performed. With respect to the aspect of polynucleotide analysis or protocol, one may use the results of the polynucleotide expression analysis or protocol to determine whether a specific therapeutic regimen should be performed.

As used herein, the term "hazard ratio" ("HR") refers to an estimate of the ratio of the hazard rate or risk rate in one group versus that in a second group. The term"hazard ratio" ("HR") is a survival analysis in the effect of an explanatory variable on the hazard or risk of an event (e.g. recurrence of disease or death). In another aspect, "hazard ratio" is an estimate of relative risk, which is the risk of an event or development of a disease relative to treatment and in some aspects the expression levels of the gene of interest. Statistical methods for determining hazard ratio are well known in the art. In proportional hazards regression models, the HR is the ratio of the predicted hazard for two groups (e.g. patients with two different kinds of treatment).

"Individual response" or "response" can be assessed using any endpoint indicating a benefit to the individual, including, without limitation, (1) inhibition, to some extent, of disease progression (e.g., cancer progression), including slowing down and complete arrest; (2) a reduction in tumor size; (3) inhibition (i.e., reduction, slowing down or complete stopping) of cancer cell infiltration into adjacent peripheral organs and/or tissues; (4) inhibition (i.e. reduction, slowing down or complete stopping) of metatasis; (5) relief, to some extent, of one or more symptoms associated with the disease or disorder (e.g., cancer); (6) increase or extend in the length of survival, including overall survival and progression free survival; and/or (7) decreased mortality at a given point of time following treatment.

An "effective response" of a patient or a patient's "responsiveness" to treatment with a medicament and similar wording refers to the clinical or therapeutic benefit imparted to a patient at risk for, or suffering from, a disease or disorder, such as cancer. In one embodiment, such benefit includes any one or more of: extending survival (including overall survival and progression free survival); resulting in an objective response (including a complete response or a partial response); or improving signs or symptoms of cancer. In one embodiment, the presence of the biomarker is used to identify a patient who is more likely to respond to treatment with a medicament, relative to a patient that does not have the presence of the biomarker. In another embodiment, the presence of the biomarker is used to determine that a patient will have an increase likelihood of benefit from treatment with a medicament, relative to a patient that does not have the presence of the biomarker.

"Survival" refers to the patient remaining alive, and includes overall survival as well as progression free survival.

"Overall survival" refers to the patient remaining alive for a defined period of time, such as 1 year, 5 years, etc from the time of diagnosis or treatment.

"Progression free survival" refers to the patient remaining alive, without the cancer progressing or getting worse.

By "extending survival" is meant increasing overall or progression free survival in a treated patient relative to an untreated patient (i.e. relative to a patient not treated with the medicament), or relative to a patient who does not express a biomarker at the designated level, and/or relative to a patient treated with an approved anti-tumor agent. An objective response refers to a measurable response, including complete response (CR) or partial response (PR).

By complete response or "CR" is intended the disappearance of all signs of cancer in response to treatment. This does not always mean the cancer has been cured.

Partial response or "PR" refers to a decrease in the size of one or more tumors or lesions, or in the extent of cancer in the body, in response to treatment.

An "effective amount" of an agent refers to an amount effective, at dosages and for periods of time necessary, to achieve the desired therapeutic or prophylactic result.

A "therapeutically effective amount" refers to an amount of a therapeutic agent to treat or prevent a disease or disorder in a mammal. In the case of cancers, the therapeutically effective amount of the therapeutic agent may reduce the number of cancer cells; reduce the primary tumor size; inhibit (i.e., slow to some extent and preferably stop) cancer cell infiltration into peripheral organs; inhibit (i.e., slow to some extent and preferably stop) tumor metastasis; inhibit, to some extent, tumor growth; and/or relieve to some extent one or more of the symptoms associated with the disorder. To the extent the drug may prevent growth and/or kill existing cancer cells, it may be cytostatic and/or cytotoxic. For cancer therapy, efficacy *in vivo* can, for example, be measured by assessing the duration of survival, time to disease progression (TTP), the response rates (RR), duration of response, and/or quality of life.

The terms "cancer" and "cancerous" refer to or describe the physiological condition in mammals that is typically characterized by unregulated cell growth. Included in this definition are benign and malignant cancers. By "early stage cancer" or "early stage tumor" is meant a cancer that is not invasive or metastatic or is classified as a Stage 0, I, or II cancer. Examples of cancer include, but are not limited to, carcinoma, lymphoma, blastoma (including medulloblastoma and retinoblastoma), sarcoma (including liposarcoma and synovial cell sarcoma), neuroendocrine tumors (including carcinoid tumors, gastrinoma, and islet cell cancer), mesothelioma, schwannoma (including acoustic neuroma), meningioma, adenocarcinoma, melanoma, and leukemia or lymphoid malignancies. More particular examples of such cancers include squamous cell cancer (e.g. epithelial squamous cell cancer), lung cancer including small-cell lung cancer (SCLC), non-small cell lung cancer (NSCLC), adenocarcinoma of the lung and squamous carcinoma of the lung, cancer of the peritoneum, hepatocellular cancer, gastric or stomach cancer including gastrointestinal cancer, pancreatic cancer, glioblastoma, cervical cancer, ovarian cancer, liver cancer, bladder cancer, hepatoma, breast cancer (including metastatic breast cancer), colon cancer, rectal cancer, colorectal cancer, endometrial or uterine carcinoma, salivary gland carcinoma, kidney or renal cancer, prostate cancer, vulval cancer, thyroid cancer, hepatic carcinoma, anal carcinoma, penile carcinoma, merkel cell cancer, mycoses fungoids, testicular cancer, esophageal cancer, tumors of the biliary tract, as well as head and neck cancer and hematological malignancies. In some aspects, the cancer is locally advanced or metastatic non-small cell lung cancer (NSCLC) or urothelial bladder cancer (UBC). In one specific aspect, the cancer is locally advanced or metastatic non-small cell lung cancer. In another specific aspect, the cancer is locally advanced or metastatic urothelial bladder cancer. In yet another aspect, the cancer is triple-negative metastatic breast cancer, including any histologically confirmed triple-negative (ER-, PR-, HER2-) adenocarcinoma of the breast with locally recurrent or metastatic disease (where the locally recurrent disease is not amenable to resection with curative intent).

The term "pharmaceutical formulation" refers to a preparation which is in such form as to permit the biological activity of an active ingredient contained therein to be effective, and which contains no additional components which are unacceptably toxic to a subject to which the formulation would be administered.

A "pharmaceutically acceptable carrier" refers to an ingredient in a pharmaceutical formulation, other than an active ingredient, which is nontoxic to a subject., A pharmaceutically acceptable carrier includes, but is not limited to, a buffer, excipient, stabilizer, or preservative.

As used herein, "treatment" (and grammatical variations thereof such as "treat" or "treating") refers to clinical intervention in an attempt to alter the natural course of the individual being treated, and can be performed either for prophylaxis or during the course of clinical pathology. Desirable effects of treatment include, but are not limited to, preventing occurrence or recurrence of disease, alleviation of symptoms, diminishment of any direct or indirect pathological consequences of the disease, preventing metastasis, decreasing the rate of disease progression, amelioration or palliation of the disease state, and remission or improved prognosis. In some embodiments, antibodies are used to delay development of a disease or to slow the progression of a disease.

The term "anti-cancer therapy" refers to a therapy useful in treating cancer. Examples of anti-cancer therapeutic agents include, but are limited to, e.g., chemotherapeutic agents, growth inhibitory agents, cytotoxic agents, agents used in radiation therapy, anti-angiogenesis agents, apoptotic agents, anti-tubulin agents, and other agents to treat cancer , anti-CD20 antibodies, platelet derived growth factor inhibitors (e.g., Gleevec^{™} (Imatinib Mesylate)), a COX-2 inhibitor (e.g., celecoxib), interferons, cytokines, antagonists (e.g., neutralizing antibodies) that bind to one or more of the following targets PDGFR-beta, BlyS, APRIL, BCMA receptor(s), TRAIL/Apo2, and other bioactive and organic chemical agents, etc. Combinations thereof are also included.

The term "cytotoxic agent" as used herein refers to a substance that inhibits or prevents the function of cells and/or causes destruction of cells. The term is intended to include radioactive isotopes (e.g., At211, I131, I125, Y90, Re186, Re188, Sm153, Bi212, P32 and radioactive isotopes of Lu), chemotherapeutic agents e.g., methotrexate, adriamicin, vinca alkaloids (vincristine, vinblastine, etoposide), doxorubicin, melphalan, mitomycin C, chlorambucil, daunorubicin or other intercalating agents, enzymes and fragments thereof such as nucleolytic enzymes, antibiotics, and toxins such as small molecule toxins or enzymatically active toxins of bacterial, fungal, plant or animal origin, including fragments and/or variants thereof, and the various antitumor or anticancer agents disclosed below. Other cytotoxic agents are described below. A tumoricidal agent causes destruction of tumor cells.

A "chemotherapeutic agent" refers to a chemical compound useful in the treatment of cancer. Examples of chemotherapeutic agents include alkylating agents such as thiotepa and cyclosphosphamide (CYTOXAN^{®}); alkyl sulfonates such as busulfan, improsulfan and piposulfan; aziridines such as benzodopa, carboquone, meturedopa, and uredopa; ethylenimines and methylamelamines including altretamine, triethylenemelamine, triethylenephosphoramide, triethylenethiophosphoramide and trimethylomelamine; acetogenins (especially bullatacin and bullatacinone); delta-9-tetrahydrocannabinol (dronabinol, MARINOL^{®}); beta-lapachone; lapachol; colchicines; betulinic acid; a camptothecin (including the synthetic analogue topotecan (HYCAMTIN^{®}), CPT-11 (irinotecan, CAMPTOSAR^{®}), acetylcamptothecin, scopolectin, and 9-aminocamptothecin); bryostatin; callystatin; CC-1065 (including its adozelesin, carzelesin and bizelesin synthetic analogues); podophyllotoxin; podophyllinic acid; teniposide; cryptophycins (particularly cryptophycin 1 and cryptophycin 8); dolastatin; duocarmycin (including the synthetic analogues, KW-2189 and CB1-TM1); eleutherobin; pancratistatin; a sarcodictyin; spongistatin; nitrogen mustards such as chlorambucil, chlornaphazine, chlorophosphamide, estramustine, ifosfamide, mechlorethamine, mechlorethamine oxide hydrochloride, melphalan, novembichin, phenesterine, prednimustine, trofosfamide, uracil mustard; nitrosoureas such as carmustine, chlorozotocin, fotemustine, lomustine, nimustine, and ranimnustine; antibiotics such as the enediyne antibiotics (e. g., calicheamicin, especially calicheamicin gamma1I and calicheamicin omegaI1 (see, e.g., Nicolaou et al., Angew. Chem Intl. Ed. Engl., 33: 183-186 (1994)); CDP323, an oral alpha-4 integrin inhibitor; dynemicin, including dynemicin A; an esperamicin; as well as neocarzinostatin chromophore and related chromoprotein enediyne antibiotic chromophores), aclacinomysins, actinomycin, authramycin, azaserine, bleomycins, cactinomycin, carabicin, carminomycin, carzinophilin, chromomycins, dactinomycin, daunorubicin, detorubicin, 6-diazo-5-oxo-L-norleucine, doxorubicin (including ADRIAMYCIN^{®}, morpholino-doxorubicin, cyanomorpholino-doxorubicin, 2-pyrrolino-doxorubicin, doxorubicin HCl liposome injection (DOXIL^{®}), liposomal doxorubicin TLC D-99 (MYOCET^{®}), peglylated liposomal doxorubicin (CAELYX^{®}), and deoxydoxorubicin), epirubicin, esorubicin, idarubicin, marcellomycin, mitomycins such as mitomycin C, mycophenolic acid, nogalamycin, olivomycins, peplomycin, porfiromycin, puromycin, quelamycin, rodorubicin, streptonigrin, streptozocin, tubercidin, ubenimex, zinostatin, zorubicin; anti-metabolites such as methotrexate, gemcitabine (GEMZAR^{®}), tegafur (UFTORAL^{®}), capecitabine (XELODA^{®}), an epothilone, and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; eniluracil; amsacrine; bestrabucil; bisantrene; edatraxate; defofamine; demecolcine; diaziquone; elfornithine; elliptinium acetate; an epothilone; etoglucid; gallium nitrate; hydroxyurea; lentinan; lonidainine; maytansinoids such as maytansine and ansamitocins; mitoguazone; mitoxantrone; mopidanmol; nitraerine; pentostatin; phenamet; pirarubicin; losoxantrone; 2-ethylhydrazide; procarbazine; PSK^{®} polysaccharide complex (JHS Natural Products, Eugene, OR); razoxane; rhizoxin; sizofiran; spirogermanium; tenuazonic acid; triaziquone; 2,2',2'-trichlorotriethylamine; trichothecenes (especially T-2 toxin, verracurin A, roridin A and anguidine); urethan; vindesine (ELDISINE^{®}, FILDESIN^{®}); dacarbazine; mannomustine; mitobronitol; mitolactol; pipobroman; gacytosine; arabinoside ("Ara-C"); thiotepa; taxoid, e.g., paclitaxel (TAXOL^{®}), albumin-engineered nanoparticle formulation of paclitaxel (ABRAXANETM), and docetaxel (TAXOTERE^{®}), chloranbucil; 6-thioguanine; mercaptopurine; methotrexate; platinum agents such as cisplatin, oxaliplatin (e.g., ELOXATIN^{®}), and carboplatin; vincas, which prevent tubulin polymerization from forming microtubules, including vinblastine (VELBAN^{®}), vincristine (ONCOVIN^{®}), vindesine (ELDISINE^{®}, FILDESIN^{®}), and vinorelbine (NAVELBINE^{®}); etoposide (VP-16); ifosfamide; mitoxantrone; leucovorin; novantrone; edatrexate; daunomycin; aminopterin; ibandronate; topoisomerase inhibitor RFS 2000; difluoromethylornithine (DMFO); retinoids such as retinoic acid, including bexarotene (TARGRETIN^{®}); bisphosphonates such as clodronate (for example, BONEFOS^{®} or OSTAC^{®}), etidronate (DIDROCAL^{®}), NE-58095, zoledronic acid/zoledronate (ZOMETA^{®}), alendronate (FOSAMAX^{®}), pamidronate (AREDIA^{®}), tiludronate (SKELID^{®}), or risedronate (ACTONEL^{®}); troxacitabine (a 1,3-dioxolane nucleoside cytosine analog); antisense oligonucleotides, particularly those that inhibit expression of genes in signaling pathways implicated in aberrant cell proliferation, such as, for example, PKC-alpha, Raf, H-Ras, and epidermal growth factor receptor (EGF-R); vaccines such as THERATOPE^{®} vaccine and gene therapy vaccines, for example, ALLOVECTIN^{®} vaccine, LEUVECTIN^{®} vaccine, and VAXID^{®} vaccine; topoisomerase 1 inhibitor (e.g., LURTOTECAN^{®}); rmRH (e.g., ABARELIX^{®}); BAY439006 (sorafenib; Bayer); SU-11248 (sunitinib, SUTENT^{®}, Pfizer); perifosine, COX-2 inhibitor (e.g., celecoxib or etoricoxib), proteosome inhibitor (e.g., PS341); bortezomib (VELCADE^{®}); CCI-779; tipifarnib (R11577); orafenib, ABT510; Bcl-2 inhibitor such as oblimersen sodium (GENASENSE^{®}); pixantrone; EGFR inhibitors (see definition below); tyrosine kinase inhibitors (see definition below); serine-threonine kinase inhibitors such as rapamycin (sirolimus, RAPAMUNE^{®}); farnesyltransferase inhibitors such as lonafarnib (SCH 6636, SARASARTM); and pharmaceutically acceptable salts, acids or derivatives of any of the above; as well as combinations of two or more of the above such as CHOP, an abbreviation for a combined therapy of cyclophosphamide, doxorubicin, vincristine, and prednisolone; and FOLFOX, an abbreviation for a treatment regimen with oxaliplatin (ELOXATINTM) combined with 5-FU and leucovorin.

Chemotherapeutic agents as defined herein include "anti-hormonal agents" or "endocrine therapeutics" which act to regulate, reduce, block, or inhibit the effects of hormones that can promote the growth of cancer. They may be hormones themselves, including, but not limited to: anti-estrogens with mixed agonist/antagonist profile, including, tamoxifen (NOLVADEX^{®}), 4-hydroxytamoxifen, toremifene (FARESTON^{®}), idoxifene, droloxifene, raloxifene (EVISTA^{®}), trioxifene, keoxifene, and selective estrogen receptor modulators (SERMs) such as SERM3; pure anti-estrogens without agonist properties, such as fulvestrant (FASLODEX^{®}), and EM800 (such agents may block estrogen receptor (ER) dimerization, inhibit DNA binding, increase ER turnover, and/or suppress ER levels); aromatase inhibitors, including steroidal aromatase inhibitors such as formestane and exemestane (AROMASIN^{®}), and nonsteroidal aromatase inhibitors such as anastrazole (ARIMIDEX^{®}), letrozole (FEMARA^{®}) and aminoglutethimide, and other aromatase inhibitors include vorozole (RIVISOR^{®}), megestrol acetate (MEGASE^{®}), fadrozole, and 4(5)-imidazoles; lutenizing hormone-releaseing hormone agonists, including leuprolide (LUPRON^{®} and ELIGARD^{®}), goserelin, buserelin, and tripterelin; sex steroids, including progestines such as megestrol acetate and medroxyprogesterone acetate, estrogens such as diethylstilbestrol and premarin, and androgens/retinoids such as fluoxymesterone, all transretionic acid and fenretinide; onapristone; anti-progesterones; estrogen receptor down-regulators (ERDs); anti-androgens such as flutamide, nilutamide and bicalutamide; and pharmaceutically acceptable salts, acids or derivatives of any of the above; as well as combinations of two or more of the above.

A "growth inhibitory agent" when used herein refers to a compound or composition which inhibits growth of a cell (e.g., a cell whose growth is dependent upon PD-L1 expression either *in vitro* or *in vivo*)*.* Examples of growth inhibitory agents include agents that block cell cycle progression (at a place other than S phase), such as agents that induce G1 arrest and M-phase arrest. Classical M-phase blockers include the vincas (vincristine and vinblastine), taxanes, and topoisomerase II inhibitors such as doxorubicin, epirubicin, daunorubicin, etoposide, and bleomycin. Those agents that arrest G1 also spill over into S-phase arrest, for example, DNA alkylating agents such as tamoxifen, prednisone, dacarbazine, mechlorethamine, cisplatin, methotrexate, 5-fluorouracil, and ara-C. Further information can be found in The Molecular Basis of Cancer, Mendelsohn and Israel, eds., Chapter 1, entitled "Cell cycle regulation, oncogenes, and antineoplastic drugs" by Murakami et al. (WB Saunders: Philadelphia, 1995), especially p. 13. The taxanes (paclitaxel and docetaxel) are anticancer drugs both derived from the yew tree. Docetaxel (TAXOTERE^{®}, Rhone-Poulenc Rorer), derived from the European yew, is a semisynthetic analogue of paclitaxel (TAXOL^{®}, Bristol-Myers Squibb). Paclitaxel and docetaxel promote the assembly of microtubules from tubulin dimers and stabilize microtubules by preventing depolymerization, which results in the inhibition of mitosis in cells.

By "radiation therapy" is meant the use of directed gamma rays or beta rays to induce sufficient damage to a cell so as to limit its ability to function normally or to destroy the cell altogether. It will be appreciated that there will be many ways known in the art to determine the dosage and duration of treatment. Typical treatments are given as a one time administration and typical dosages range from 10 to 200 units (Grays) per day.

An "individual" or "subject" is a mammal. Mammals include, but are not limited to, domesticated animals (e.g., cows, sheep, cats, dogs, and horses), primates (e.g., humans and non-human primates such as monkeys), rabbits, and rodents (e.g., mice and rats). In certain embodiments, the individual or subject is a human.

The term "concurrently" is used herein to refer to administration of two or more therapeutic agents, where at least part of the administration overlaps in time. Accordingly, concurrent administration includes a dosing regimen when the administration of one or more agent(s) continues after discontinuing the administration of one or more other agent(s).

By "reduce or inhibit" is meant the ability to cause an overall decrease of 20%, 30%, 40%, 50%, 60%, 70%, 75%, 80%, 85%, 90%, 95%, or greater. Reduce or inhibit can refer to the symptoms of the disorder being treated, the presence or size of metastases, or the size of the primary tumor.

It is understood that the singular form "a", "an", and "the" includes plural references unless indicated otherwise.

### Stem cell maintenance-related genes

Abundance of stem cell maintenance-related genes was found to be predictive of response to PD-1 axis inhibitors. Abundance of stem cell maintenance-related genes can be determined by detecting expression levels of genes associated with stem cell phenotypes. Those markers include ASPM, CNOT3, LRP5 and PBX1. These markers may be considered as a cumulative stem cell maintenance-related gene score. The expression levels of four or more markers can be combined by any appropriate state of the art mathematical method to obtain a stem cell maintenance-related genes score. In one aspect, a stem cell maintenance-related gene score can be obtained on the basis of expression levels of genes consisting of ASPM, CNOT3, LRP5 and PBX1.

In one aspect, the biomarker of the present invention is used for predicting response of patients with renal cell carcinoma to an anti-PD-L1 antibody atezolizumab. In another embodiment, the biomarker of the present invention is used for predicting response of patients with non-small cell lung cancer (NSCLC) to an anti-PD-L1 antibody, for example atezolizumab. The predictive value is higher in patients who are PD-L1 positive. Therefore, in one aspect, the biomarker is used for predicting response of patients who are PD-L1 positive, more specifically patients with NSCLC who are PD-L1 positive, to a PD-1 axis inhibitor such as an anti-PD-L1 antibody atezolizumab. In another aspect, the biomarker is used for predicting response of patients having cancer to a PD-1 axis inhibitor such as an anti-PD-L1 antibody, for example atezolizumab.

The invention thus relates to an *in vitro* method of identifying a patient having cancer who is responsive to a therapy comprising an effective amount of an anti-PD-L1 antibody the method comprising determing the abundance of stem cell maintenance-related genes in a tumor tissue sample obtained from a patient having cancer, wherein the abundance of stem cell maintenance-related genes is characterized by detecting the expression level of ASPM, CNOT3, LRP5 and PBX1. In one aspect, the expression level of the genes selected from a group consisting of ASPM, CNOT3, LRP5 and PBX1 is detected. In one aspect, the expression level of all genes selected from a group consisting of ASPM, CNOT3, LRP5 and PBX1 is detected. In some aspects, the method comprises a step of comparing the expression level of the one or more genes to a reference level, whereby an increased expression level is indicative of response to a therapy comprising an effective amount an anti-PD-L1 antibody. In one aspect, an increased expression level is indicative of a reduced response to a therapy comprising an effective amount of an anti-PD-L1 antibody.

### Exemplary PD-1 Axis Inhibitors for use

By way of example, a PD-1 axis inhibitor includes a PD-1 binding antagonist and a PD-L1 binding antagonist. Alternative names for "PD-1" include CD279 and SLEB2. Alternative names for "PD-L1" include B7-H1, B7-4, CD274, and B7-H. In some aspects, PD-1 and PD-L1 are human PD-1 and PD-L1.

In some aspects, the PD-1 binding antagonist is a molecule that inhibits the binding of PD-1 to its ligand binding partners. In a specific aspect, the PD-1 ligand binding partners are PD-L1 and/or PDL2. In another aspect, a PDL1 binding antagonist is a molecule that inhibits the binding of PD-L1 to its binding partners. In a specific aspect, PD-L1 binding partners are PD-1 and/or B7-1. The antagonist may be an antibody, an antigen binding fragment thereof, an immunoadhesin, a fusion protein, or oligopeptide.

In some aspects, the PD-1 binding antagonist is an anti-PD-1 antibody (e.g., a human antibody, a humanized antibody, or a chimeric antibody). In some aspects, the anti-PD-1 antibody is selected from the group consisting of nivolumab, pembrolizumab, cemiplimab, pidilizumab, spartalizumab, camrelizumab and sintilimab. In some aspects, the anti-PD-1 antibody is selected from the group consisting of nivolumab and pembrolizumab. In some aspects, the anti-PD-1 antibody is In some aspects, the PD-1 binding antagonist is an immunoadhesin (e.g., an immunoadhesin comprising an extracellular or PD-1 binding portion of PD-L1 or PD-L2 fused to a constant region (e.g., an Fc region of an immunoglobulin sequence).

In one specific aspect, a PD-1 binding antagonist is nivolumab (BMS-936558 or MDX-1106). In another specific aspect, a PD-1 binding antagonist is pembrolizumab (MK03745). In one further specific aspect, a PD-1 binding antagonist is cemiplimab (REGN-2810). In one specific aspect, a PD-1 binding antagonist is spartalizumab (PDR001). In one further specific aspect, a PD-1 binding antagonist is camrelizumab (SHR1210). In one specific aspect, a PD-1 binding antagonist is sintilimab (IBI308). In one further specific aspect, a PD-1 binding antagonist is pidilizumab (CT-011). In one further specific aspect, the PD-1 antagonist is BGB-108 or BGB-A317. In one further specific aspect, the PD-1 antagonist is TSR-042 (ANB011). In one further specific aspect, the PD-1 antagonist is PF-06801591 (sasanlimab). In another specific aspect, a PD-1 binding antagonist is PD1-0103 or humanized versions thereof as described in WO 2017/055443 A1.

Nivolumab, also known as MDX-1106-04, MDX-1106, ONO-4538, BMS-936558, and OPDIVO^{®}, is an anti-PD-1 antibody described in WO2006/121168. Pembrolizumab, also known as MK-3475, Merck 3475, lambrolizumab, KEYTRUDA^{®}, and SCH-900475, is an anti-PD-1 antibody described in WO2009/114335. In some aspects, the PD-1 binding antagonist is AMP-224. AMP-224, also known as B7-DCIg, is a PD-L2-Fc fusion soluble receptor described in WO2010/027827 and WO2011/066342.

In some aspects, the PD-L1 binding antagonist is anti-PD-L1 antibody. In some aspects, the PD-1 axis binding antagonist is an anti-PD-L1 antibody. In some aspects, the anti-PD-L1 antibody is capable of inhibiting binding between PD-L1 and PD-1 and/or between PD-L1 and B7-1. In some embodiments, the anti-PD-L1 antibody is a monoclonal antibody. In some aspects, the anti-PD-L1 antibody is an antibody fragment selected from the group consisting of Fab, Fab'-SH, Fv, scFv, and (Fab')₂ fragments. In some aspects, the anti-PDL1 antibody is a humanized antibody. In some aspects, the anti-PDL1 antibody is a human antibody.

In some aspects, the anti-PDL1 binding antagonist is selected from the group consisting of atezolizumab, avelumab, durvalumab and MDX-1105. MDX-1105, also known as BMS-936559, is an anti-PDL1 antibody described in WO2007/005874. Atezolizumab is an anti-PDL1 antibody described in WO 2010/077634 A1. Durvalumab (MEDI4736) is an anti-PDL1 antibody described in WO2011/066389 and US2013/034559. Avelumab (PF-06834635) is an anti-PDL1 antibody described in WO 2013/079174.

Examples of anti-PD-L1 antibodies useful for the methods of this invention, and methods for making thereof are described in PCT patent application WO 2010/077634 A1 and US Patent No. 8,217,149.

In one specific aspect, the anti-PD-L1 antibody is atezolizumab (CAS Registry Number: 1422185-06-5). Atezolizumab (Genentech), also known as MPDL3280A, is an anti-PD-L1 antibody.

Atezolizumab comprises:
(a) an HVR-H1, HVR-H2, and HVR-H3 sequence of GFTFSDSWIH (SEQ ID NO:1), AWISPYGGSTYYADSVKG (SEQ ID NO:2) and RHWPGGFDY (SEQ ID NO:3), respectively, and (b) an HVR-L1, HVR-L2, and HVR-L3 sequence of RASQDVSTAVA (SEQ ID NO:4), SASFLYS (SEQ ID NO:5) and QQYLYHPAT (SEQ ID NO:6), respectively.

Atezolizumab comprises a heavy chain and a light chain sequence, wherein:
(a) the heavy chain variable region sequence comprises the amino acid sequence: 


 (SEQ ID NO:7),
 and
(b) the light chain variable region sequence comprises the amino acid sequence:

DIQMTQSPSSLSASVGDRVTITCRASQDVSTAVAWYQQKPGKAPKLLIYSASF
LYSGVPSRFSGSGSGTDFTLTISSLQPEDFATYYCQQYLYHPATFGQGTKVEIKR (SEQ ID
NO:8).

Atezolizumab comprises a heavy chain and a light chain sequence, wherein:
(a) the heavy chain comprises the amino acid sequence: 
EVQLVESGGGLVQPGGSLRLSCAASGFTFSDSWIHWVRQAPGKGLEWVAWISPYGGST
YYADSVKGRFTISADTSKNTAYLQMNSLRAEDTAVYYCARRHWPGGFDYWGQGTLVT
VSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKKVEPKSCDKTHTCPPCPAPEL
LGGPSVFLFPPKPKDTLMISRTPEVTCVVVDVSHEDPEVKFNWYVDGVEVHNAKTKPRE
EQYASTYRVVSVLTVLHQDWLNGKEYKCKVSNKALPAPIEKTISKAKGQPREPQVYTLP
PSREEMTKNQVSLTCLVKGFYPSDIAVEWESNGQPENNYKTTPPVLDSDGSFFLYSKLT
VDKSRWQQGNVFSCSVMHEALHNHYTQKSLSLSPG (SEQ ID NO:9),
 and
(b) the light chain comprises the amino acid sequence:

DIQMTQSPSSLSASVGDRVTITCRASQDVSTAVAWYQQKPGKAPKLLIYSASFLYSGVPS
RFSGSGSGTDFTLTISSLQPEDFATYYCQQYLYHPATFGQGTKVEIKRTVAAPSVFIFPPS
DEQLKSGTASVVCLLNNFYPREAKVQWKVDNALQSGNSQESVTEQDSKDSTYSLSSTL
TLSKADYEKHKVYACEVTHQGLSSPVTKSFNRGEC (SEQ ID NO:10).

The antibody or antigen binding fragment thereof, may be made using methods known in the art, for example, by a process comprising culturing a host cell containing nucleic acid encoding any of the previously described anti-PD-L1, anti-PD-1, or anti-PD-L2 antibodies or antigen-binding fragment in a form suitable for expression, under conditions suitable to produce such antibody or fragment, and recovering the antibody or fragment.

In any of the embodiments herein, the isolated anti-PDL1 antibody can bind to a human PDL1, for example a human PDL1 as shown in UniProtKB/Swiss-Prot Accession No.Q9NZQ7.1, or a variant thereof.

In a still further aspect, provided is a composition comprising an anti-PD-L1, an anti-PD-1, or an anti-PD-L2 antibody or antigen binding fragment thereof as provided herein and at least one pharmaceutically acceptable carrier. In one aspect, provided is a pharmaceutical composition comprising an anti-PD-L1, an anti-PD-1, or an anti-PD-L2 antibody or antigen binding fragment thereof for use in the treatment of a patient having cancer, wherein the patient is determined to be responsive to a therapy comprising an effective amount of an anti-PD-L1, an anti-PD-1, or an anti-PD-L2 antibody or antigen binding fragment thereof in accordance with the method described herein. In some aspects, the anti-PD-L1, anti-PD-1, or anti-PD-L2 antibody or antigen binding fragment thereof administered to the individual is a composition comprising one or more pharmaceutically acceptable carrier.

In some aspects, the anti-PD-L1 antibody described herein is in a formulation comprising the antibody at an amount of about 60 mg/mL, histidine acetate in a concentration of about 20 mM, sucrose in a concentration of about 120 mM, and polysorbate (e.g., polysorbate 20) in a concentration of 0.04% (w/v), and the formulation has a pH of about 5.8. In some embodiments, the anti-PD-L1 antibody described herein is in a formulation comprising the antibody in an amount of about 125 mg/mL, histidine acetate in a concentration of about 20 mM, sucrose is in a concentration of about 240 mM, and polysorbate (e.g., polysorbate 20) in a concentration of 0.02% (w/v), and the formulation has a pH of about 5.5.

### Assays

In some aspects, the biomarker is detected in the sample using a method selected from the group consisting of FACS, Western blot, ELISA, immunoprecipitation, immunohistochemistry, immunofluorescence, radioimmunoassay, immunodetection methods, mass spectrometery, qPCR, RT-qPCR, multiplex qPCR or RT-qPCR, RNA-seq, microarray analysis, nanostring, SAGE, MassARRAY technique, and FISH, and combinations thereof. In some embodiments, the biomarker is detected in the sample by protein expression. In some embodiments, protein expression is determined by immunohistochemistry (IHC).

In some aspects, the biomarker is detected in the sample by mRNA expression. In some embodiments, the mRNA expression is determined using qPCR, rtPCR, RNA-seq, multiplex qPCR or RT-qPCR, microarray analysis, nanostring, SAGE, MassARRAY technique, or FISH.

In some aspects, the sample is a tumor tissue sample. In some embodiments, the tumor tissue sample comprises tumor cells, tumor infiltrating immune cells, stromal cells or any combinations thereof.

In some aspects, the sample is obtained prior to treatment with a PD-L1 axis inhibitor. In some embodiments, the tissue sample is formalin fixed and paraffin embedded, archival, fresh or frozen.

Presence and/or expression level/amount of various biomarkers in a sample can be analyzed by a number of methodologies, many of which are known in the art and understood by the skilled artisan, including, but not limited to, immunohistochemistry ("IHC"), Western blot analysis, immunoprecipitation, molecular binding assays, ELISA, ELIFA, fluorescence activated cell sorting ("FACS"), MassARRAY, proteomics, quantitative blood based assays (as for example Serum ELISA), biochemical enzymatic activity assays, in situ hybridization, Southern analysis, Northern analysis, whole genome sequencing, polymerase chain reaction ("PCR") including quantitative real time PCR ("qRT-PCR") and other amplification type detection methods, such as, for example, branched DNA, SISBA, TMA and the like), RNA-Seq, FISH, microarray analysis, gene expression profiling, and/or serial analysis of gene expression ("SAGE"), as well as any one of the wide variety of assays that can be performed by protein, gene, and/or tissue array analysis. Typical protocols for evaluating the status of genes and gene products are found, for example in Ausubel et al., eds., 1995, Current Protocols In Molecular Biology, Units 2 (Northern Blotting), 4 (Southern Blotting), 15 (Immunoblotting) and 18 (PCR Analysis). Multiplexed immunoassays such as those available from Rules Based Medicine or Meso Scale Discovery ("MSD") may also be used.

In one aspect, the sample is a clinical sample. In another embodiment, the sample is used in a diagnostic assay. In some aspects, the sample is obtained from a primary or metastatic tumor. Tissue biopsy is often used to obtain a representative piece of tumor tissue.

In certain aspects, a reference sample, reference tissue, control sample, or control tissue is a single sample or combined multiple samples from the same subject or individual that are obtained at one or more different time points than when the test sample is obtained. In certain aspects, a reference sample, reference tissue, control sample, or control tissue is a combined multiple samples from one or more healthy individuals who are not the subject or individual. In certain aspects, a reference sample, reference tissue, control sample, or control tissue is a combined multiple samples from one or more individuals with a disease or disorder (e.g., cancer) who are not the subject or individual.

In some aspects, the sample is a tumor tissue sample (e.g., biopsy tissue). In some aspects, the tissue sample is lung tissue. In some aspects, the tissue sample is renal tissue. In some aspects, the tissue sample is skin tissue. In some aspects, the tissue sample is pancreatic tissue. In some aspects, the tissue sample is gastric tissue. In some embodiments, the tissue sample is bladder tissue. In some aspects, the tissue sample is esophageal tissue. In some aspects, the tissue sample is mesothelial tissue. In some aspects, the tissue sample is breast tissue. In some aspects, the tissue sample is thyroid tissue. In some aspects, the tissue sample is colorectal tissue. In some aspects, the tissue sample is head and neck tissue. In some aspects, the tissue sample is osteosarcoma tissue. In some aspects, the tissue sample is prostate tissue. In some aspects, the tissue sample is ovarian tissue, HCC (liver), blood cells, lymph nodes, bone/bone marrow.

### Therapeutic methods

Provided are methods for treating cancer in an individual, the method comprising: determining the abundance of stem cell maintenance-related genes in a tumor tissue sample from the individual, and administering an effective amount of a PD-1 axis inhibitor to the individual.

In some aspects, an increased expression of biomarkers related to stem cell maintenance-indicates that the individual is more likely to have increased clinical benefit when the individual is treated with the PD-L1 axis inhibitor. In some aspects, the increased clinical benefit comprises a relative increase in one or more of the following: overall survival (OS), progression free survival (PFS), complete response (CR), partial response (PR) and combinations thereof.

A PD-1 axis inhibitor described herein can be used either alone or in combination with other agents in a therapy. For instance, a PD-1 axis inhibitor described herein may be co-administered with at least one additional therapeutic agent. In certain aspects, an additional therapeutic agent is a chemotherapeutic agent.

Such combination therapies noted above encompass combined administration (where two or more therapeutic agents are included in the same or separate formulations), and separate administration, in which case, administration of the antagonist can occur prior to, simultaneously, and/or following, administration of the additional therapeutic agent and/or adjuvant. PD-1 axis inhibitor described herein can also be used in combination with radiation therapy.

A PD-1 axis inhibitor (e.g., an antibody, binding polypeptide, and/or small molecule) described herein (and any additional therapeutic agent) can be administered by any suitable means, including parenteral, intrapulmonary, and intranasal, and, if desired for local treatment, intralesional administration. Parenteral infusions include intramuscular, intravenous, intraarterial, intraperitoneal, or subcutaneous administration. Dosing can be by any suitable route, e.g., by injections, such as intravenous or subcutaneous injections, depending in part on whether the administration is brief or chronic. Various dosing schedules including but not limited to single or multiple administrations over various time-points, bolus administration, and pulse infusion are contemplated herein.

A PD-1 axis inhibitor (e.g., an antibody, binding polypeptide, and/or small molecule) described herein may be formulated, dosed, and administered in a fashion consistent with good medical practice. Factors for consideration in this context include the particular disorder being treated, the particular mammal being treated, the clinical condition of the individual patient, the cause of the disorder, the site of delivery of the agent, the method of administration, the scheduling of administration, and other factors known to medical practitioners. The PD-1 axis inhibitor need not be, but is optionally formulated with one or more agents currently used to prevent or treat the disorder in question. The effective amount of such other agents depends on the amount of the PD-1 axis inhibitor present in the formulation, the type of disorder or treatment, and other factors discussed above. These are generally used in the same dosages and with administration routes as described herein, or about from 1 to 99% of the dosages described herein, or in any dosage and by any route that is empirically/clinically determined to be appropriate.

For the prevention or treatment of disease, the appropriate dosage of a PD-1 axis inhibitor described herein (when used alone or in combination with one or more other additional therapeutic agents) will depend on the type of disease to be treated, the severity and course of the disease, whether the PD-1 axis inhibitor is administered for preventive or therapeutic purposes, previous therapy, the patient's clinical history and response to the PD-1 axis inhibitor, and the discretion of the attending physician. The PD-1 axis inhibitor is suitably administered to the patient at one time or over a series of treatments. One typical daily dosage might range from about 1 µg/kg to 100 mg/kg or more, depending on the factors mentioned above. For repeated administrations over several days or longer, depending on the condition, the treatment would generally be sustained until a desired suppression of disease symptoms occurs. Such doses may be administered intermittently, e.g., every week or every three weeks (e.g., such that the patient receives from about two to about twenty, or e.g., about six doses of the PD-1 axis inhibitor). An initial higher loading dose, followed by one or more lower doses may be administered. An exemplary dosing regimen comprises administering. However, other dosage regimens may be useful. The progress of this therapy is easily monitored by conventional techniques and assays.

In some aspects, the PD-1 axis inhibitor (e.g., anti- PD-L1 antibody) is administered at a dosage of about 0.3-30 mg/kg. In some embodiments, the PD-L1 axis binding antagonist (e.g., anti- PD-L1 antibody) is administered at a dosage of about any of 0.3 mg/kg, 0.5 mg/kg, 1 mg/kg, 2 mg/kg, 4 mg/kg, 8 mg/kg, 15 mg/kg, 20 mg/kg, or 30 mg/kg. In some embodiments, the PD-1 axis inhibitor (e.g., anti- PD-L1 antibody) is administered at a dosage of about any of 2 mg/kg, 4 mg/kg, 8 mg/kg, 15 mg/kg, or 30 mg/kg in 21-day cycles. It is understood that any of the above formulations or therapeutic methods may be carried out using an immunoconjugate in place of or in addition to the PD-1 axis inhibitor.

Pharmaceutical formulations of a PD-1 axis inhibitor as described herein are prepared by mixing such antibody having the desired degree of purity with one or more optional pharmaceutically acceptable carriers (Remington's Pharmaceutical Sciences 16th edition, Osol, A. Ed. (1980)), in the form of lyophilized formulations or aqueous solutions. In some embodiments, the PD-1 axis inhibitor is a binding small molecule, an antibody, binding polypeptide, and/or polynucleotide. Pharmaceutically acceptable carriers are generally nontoxic to recipients at the dosages and concentrations employed, and include, but are not limited to: buffers such as phosphate, citrate, and other organic acids; antioxidants including ascorbic acid and methionine; preservatives (such as octadecyldimethylbenzyl ammonium chloride; hexamethonium chloride; benzalkonium chloride; benzethonium chloride; phenol, butyl or benzyl alcohol; alkyl parabens such as methyl or propyl paraben; catechol; resorcinol; cyclohexanol; 3-pentanol; and m-cresol); low molecular weight (less than about 10 residues) polypeptides; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as polyethylene glycol (PEG). Exemplary pharmaceutically acceptable carriers herein further include insterstitial drug dispersion agents such as soluble neutral-active hyaluronidase glycoproteins (sHASEGP), for example, human soluble PH-20 hyaluronidase glycoproteins, such as rHuPH20 (HYLENEX^{®}, Baxter International, Inc.). Certain exemplary sHASEGPs and methods of use, including rHuPH20, are described in US Patent Publication Nos. 2005/0260186 and 2006/0104968. In one embodiment, a sHASEGP is combined with one or more additional glycosaminoglycanases such as chondroitinases.

Exemplary lyophilized formulations are described in US Patent No. 6,267,958. Aqueous antibody formulations include those described in US Patent No. 6,171,586 and WO2006/044908, the latter formulations including a histidine-acetate buffer.

The formulation herein may also contain more than one active ingredients as necessary for the particular indication being treated, preferably those with complementary activities that do not adversely affect each other. Such active ingredients are suitably present in combination in amounts that are effective for the purpose intended.

Active ingredients may be entrapped in microcapsules prepared, for example, by coacervation techniques or by interfacial polymerization, for example, hydroxymethylcellulose or gelatin-microcapsules and poly-(methylmethacylate) microcapsules, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980).

Sustained-release preparations may be prepared. Suitable examples of sustained-release preparations include semipermeable matrices of solid hydrophobic polymers containing the PD-L1 axis binding antagonist, which matrices are in the form of shaped articles, e.g., films, or microcapsules.

The formulations to be used for *in vivo* administration are generally sterile. Sterility may be readily accomplished, e.g., by filtration through sterile filtration membranes.

### Diagnostic kits, assays and articles of manufacture

Provided herein are diagnostic kits comprising one or more reagent for determining the presence of a biomarker in a sample from an individual with a disease or disorder.

Provided herein are also assays for identifying an individual with a disease or disorder to receive a PD-L1 axis inhibitor, the method comprising: determining the abundance of stem cell maintenance-related genes in a tumor tissue sample from the individual, and recommending a PD-1 axis inhibitor based on the abundance of stem cell maintenance-related genes.

Provided herein are also articles of manufacture comprising, packaged together, a PD-1 axis inhibitor (e.g., anti- PD-L1 antibodies) in a pharmaceutically acceptable carrier and a package insert indicating that the PD-L1 axis inhibitor (e.g., anti- PD-L1 antibodies) is for treating a patient with a disease or disorder based on abundance of stem cell maintenance-related genes or expression levels biomarkers related to development of stem cell maintenance-related genes. Treatment methods include any of the treatment methods disclosed herein. Further provided are a method for manufacturing an article of manufacture comprising combining in a package a pharmaceutical composition comprising a PD-1 axis inhibitor (e.g., anti- PD-L1 antibodies) and a package insert indicating that the pharmaceutical composition is for treating a patient with a disease or disorder based on abundance of stem cell maintenance-related genes or expression levels of biomarkers related to development of stem cells.

The article of manufacture comprises a container and a label or package insert on or associated with the container. Suitable containers include, for example, bottles, vials, syringes, etc. The containers may be formed from a variety of materials such as glass or plastic. The container holds or contains a composition comprising the cancer medicament as the active agent and may have a sterile access port (for example the container may be an intravenous solution bag or a vial having a stopper pierceable by a hypodermic injection needle).

The article of manufacture may further comprise a second container comprising a pharmaceutically-acceptable diluent buffer, such as bacteriostatic water for injection (BWFI), phosphate-buffered saline, Ringer's solution and dextrose solution. The article of manufacture may further include other materials desirable from a commercial and user standpoint, including other buffers, diluents, filters, needles, and syringes.

The article of manufacture also includes information, for example in the form of a package insert, indicating that the composition is used for treating cancer based on expression level of the biomarker(s) herein. The insert or label may take any form, such as paper or on electronic media such as a magnetically recorded medium (e.g., floppy disk) or a CD-ROM. The label or insert may also include other information concerning the pharmaceutical compositions and dosage forms in the kit or article of manufacture.
The present invention is further described by reference to the following non-limited figures and examples.

### Examples

### Example 1

### Stemness-related gene transcripts predict clinical benefit in patients with Locally Advanced or Metastatic Non-Small Cell Lung Cancer treated with atezolizumab

We hypothesized that patients with a low abundance of stem cell maintenance-related cancer cells may respond to PD-L1 blockade leading to beneficial effect in patients who received the treatment. We analyzed 739 patients with non-small cell lung cancer who received Atezolizumab (MPDL3280A, n=358) and Docetaxel (n=381) in a Phase III clinical trial (NCT02008227, OAK) (https://clinicaltrials.gov/ct2/show/NCT02008227). This study was sponsored by Genentech Inc., a member of the Roche Group, which provided the study drug. The protocol and its amendments were approved by the relevant institutional review boards or ethics committees, and all participants provided written informed consent. This study was conducted in accordance with the Declaration of Helsinki and International Conference on Harmonization Guidelines for Good Clinical Practice.

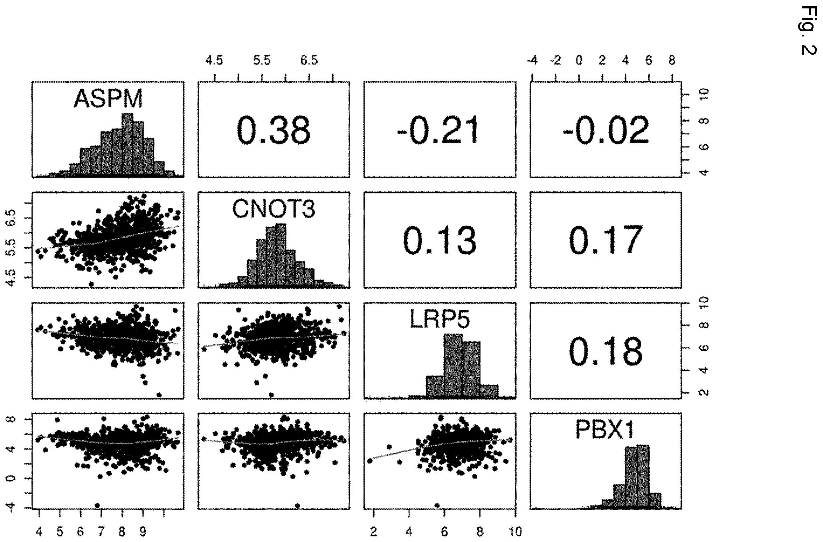

Tumor specimens at baseline were archived and taken for gene expression profiling performed by RNA Sequencing (RNA-Seq). In total, 144 human stem cell related genes derived from Gene Ontology were selected and further processed resulting in a selected a list of 4 genes consisting of ASPM, CNOT3, LRP5, and PBX1, which are associated with abundance of stem cell maintenance-related cancer cells. The log2 CPM expression values of each selected gene across the whole cohort were divided at medium expression level for the higher expression ones (+) and lower/no expression ones (-). Using in-house R scripts, the two defined subgroups were plotted against the Kaplan-Meier survival curves. **Figure 2** shows the correlation of single gene expression pattern with the patient survival.

**Table 1** summarizes the median survival as well as HR per single gene used for the stem cell maintenance-related cancer cells signature.

**Table 1: Single gene association with the survival advantages by a PD-1 axis inhibitor atezolizumab**

| **Gene** | **Treatment** | **Signature** | **Median survival (days)** | **HR (low vs high)** | **HR (Atezo vs SoC)** |
|---|---|---|---|---|---|
| ASPM | Docetaxel | Stem cell score high | 270 | | |
| | | Stem cell score low | 365 | | HR: 0.84 |
| | | | | | pval: 0.2261 |
| | Atezolizumab | Stem cell score low | 467 | HR: 0.65 | |
| | | Stem cell score high | 256 | pval: 0.00592 | |
| CNOT3 | Docetaxel | Stem cell score high | 299 | | |
| | | Stem cell score low | 339 | | HR: 0.83 |
| | Atezolizumab | Stem cell score low | 433 | HR: 0.89 | pval: 0.18 |
| | | Stem cell score high | 337 | pval: 0.40 | |
| LRP5 | Docetaxel | Stem cell score high | 320 | | |
| | | Stem cell score low | 312 | | HR: 0.83 |
| | Atezolizumab | Stem cell score low | 405 | HR: 0.97 | pval: 0.15 |
| | | Stem cell score high | 384 | pval: 0.83 | |
| PBX1 | Docetaxel | Stem cell score high | 262 | | |
| | | Stem cell score low | 356 | | HR: 0.92 |
| | Atezolizumab | Stem cell score low | 456 | HR: 0.89 | pval: 0.53 |
| | | Stem cell score high | 329 | pval: 0.40 | |

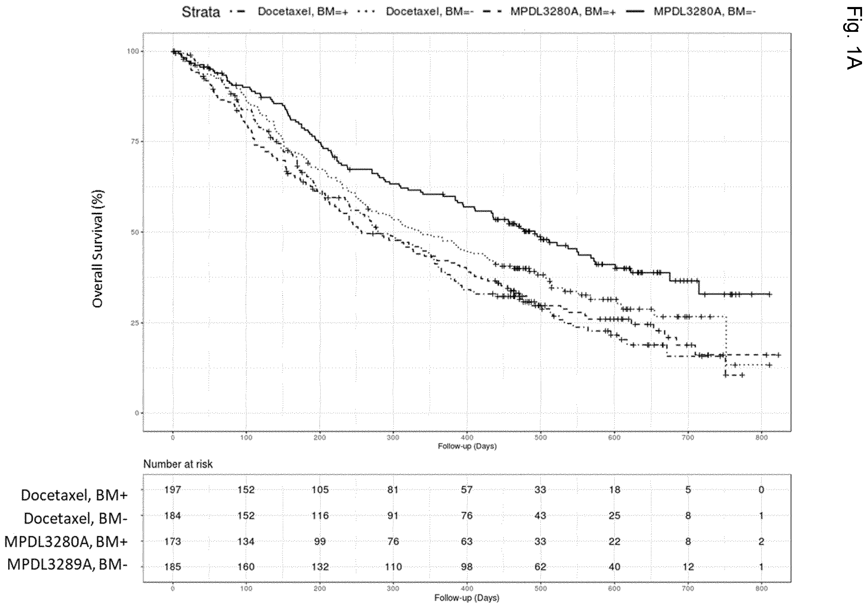

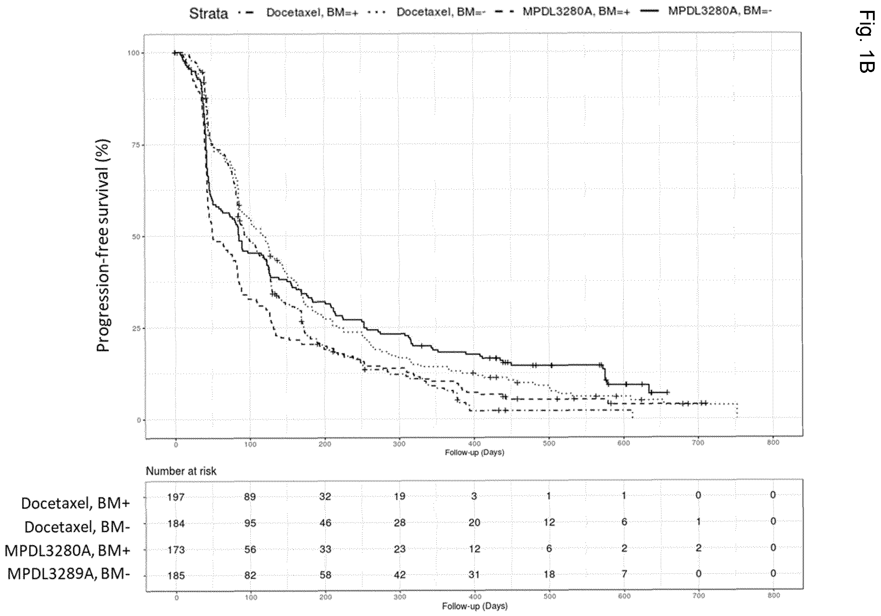

As several genes linked to stem cell maintenance-related cancer cells were associated with the survival advantages, we investigated the impact of multiple genes involved in stem cell maintenance-related cancer cells by defining a cumulative cancer stem cell gene score reflecting the cumulative expression of these marker genes. Each gene's expression is first standardized by a z-score: $z = \frac{x - μ}{σ} ,$ where µand µ are estimated in the entire cohort or in the selected subgroups. After the standardization step, these standardized z-score values are averaged across genes within each patient. The OAK cohort was corrected for the sex of patient, age, histolog as well as PD-L1 status. Based on such a analysis, we observed that patients treated with Atezolizumab and a lower cancer stem cell score showed superior overall survival advantage, having a medium overall survival of ~ 492 days, whereas the high expression group had a medium overall survival of ~257 days (HR=0.67, and p=0.005) (**Figure 1A**). As comparison, for patients having a low cancers stem cell score, the Docetaxel subgroup shows a median overall survival of 334 days (high subgroup 282 days) resulting in a HR of 0.76 and p=0.05. Therefore, the stem cell score low group shows a predictive effect for Atezolizumab (HR=0.72, and p=0.019). In addition, we also identified an advantage in progression-free survival. Patients treated with Atezolizumab and a lower cancers stem cell score showed a median progression-free survival of ~86 days and the high group of ~51 days resulting in an HR of 0.69 and p=0.001 (**Figure 1B**).

### Example 2

### Stemness-related gene transcripts predict clinical benefit in patients with Positive Locally Advanced or Metastatic Non-Small Cell Lung Cancer treated with atezolizumab

We hypothesized that patients with a low abundance of stem cell maintenance-related cancer cells may respond to PD-L1 blockade leading to beneficial effect in patients who received the treatment. We analyzed **597** patients with non-small cell lung cancer who received Atezolizumab in a Phase II clinical trial (NCT02031458, BIRCH) (https://clinicaltrials.gov/ct2/ show/NCT02031458). This study was sponsored by Genentech Inc., a member of the Roche Group, which provided the study drug. The protocol and its amendments were approved by the relevant institutional review boards or ethics committees, and all participants provided written informed consent. This study was conducted in accordance with the Declaration of Helsinki and International Conference on Harmonization Guidelines for Good Clinical Practice.

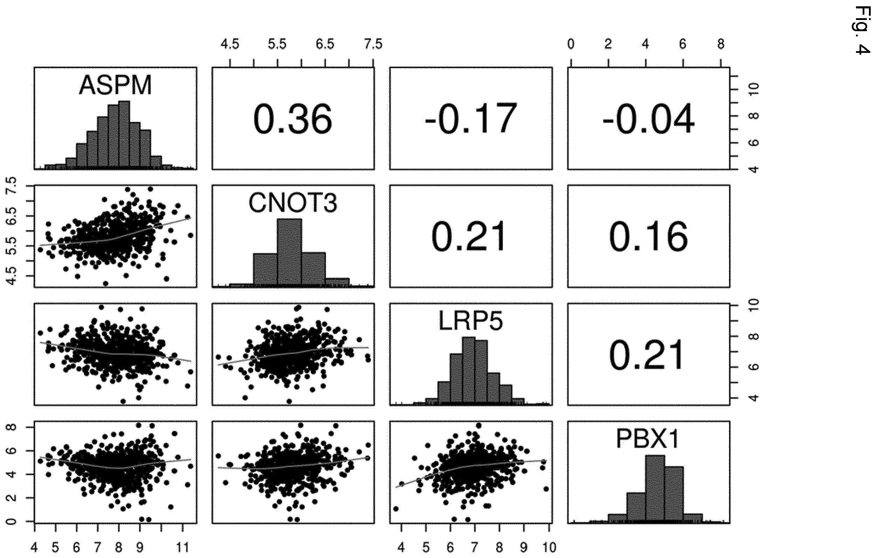

Tumor specimens at baseline were archived and taken for gene expression profiling performed by RNA Sequencing (RNA-Seq). ). In total, 144 human stem cell related genes derived from Gene Ontology were selected and further processed resulting in a selected a list of 4 genes consisting of ASPM, CNOT3, LRP5, and PBX1, which are associated with abundance of stem cell maintenance-related cancer cells. The log2 CPM expression values of each selected gene across the whole cohort were divided at medium expression level for the higher expression ones (+) and lower/no expression ones (-). Using in-house R scripts, the two defined subgroups were plotted against the Kaplan-Meier survival curves. **Figure 4** shows the correlation of single gene expression pattern with the patient survival. **Table 2** summarizes the median survival as well as HR per single gene used for the stem cell maintenance-related cancer cells signature.

**Table 2: single gene association with the survival advantages by a PD-1 axis inhibitor Atezolizumab**

| **Gene** | **Treatment** | **Signature** | **Median survival (days)** | **HR (low vs high)** |
|---|---|---|---|---|
| ASPM | Atezolizumab | Stem cell score low | 566 | HR: 0.78 |
| | | Stem cell score high | 457 | pval : 0.01 |
| CNOT3 | Atezolizumab | Stem cell score low | 510 | HR: 0.88 |
| | | Stem cell score high | 482 | pval: 0.19 |
| LRP5 | Atezolizumab | Stem cell score low | 471 | HR: 1.02 |
| | | Stem cell score high | 498 | pval: 0.87 |
| PBX1 | Atezolizumab | Stem cell score low | 471 | HR: 1.02 |
| | | Stem cell score high | 510 | pval: 0.87 |

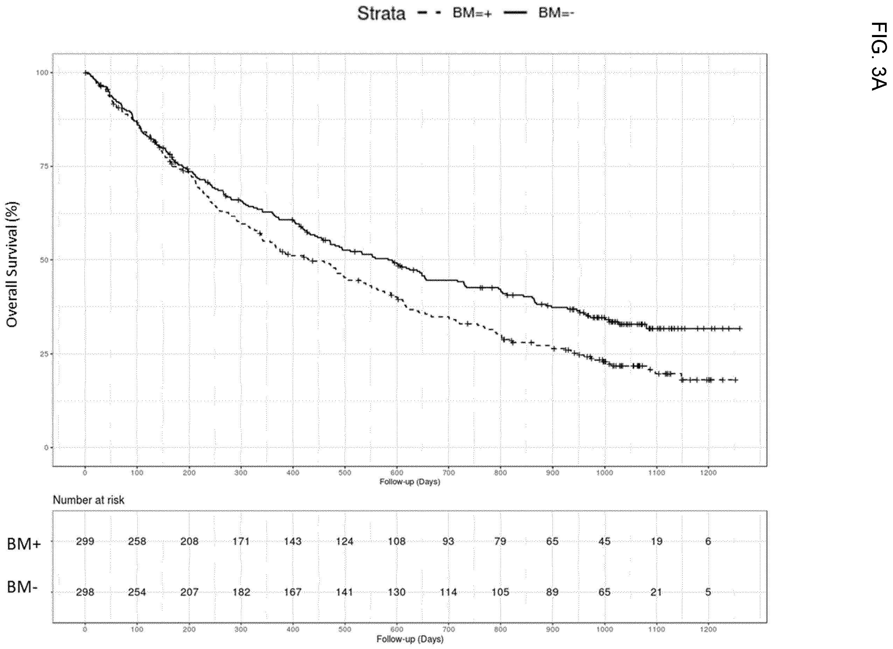

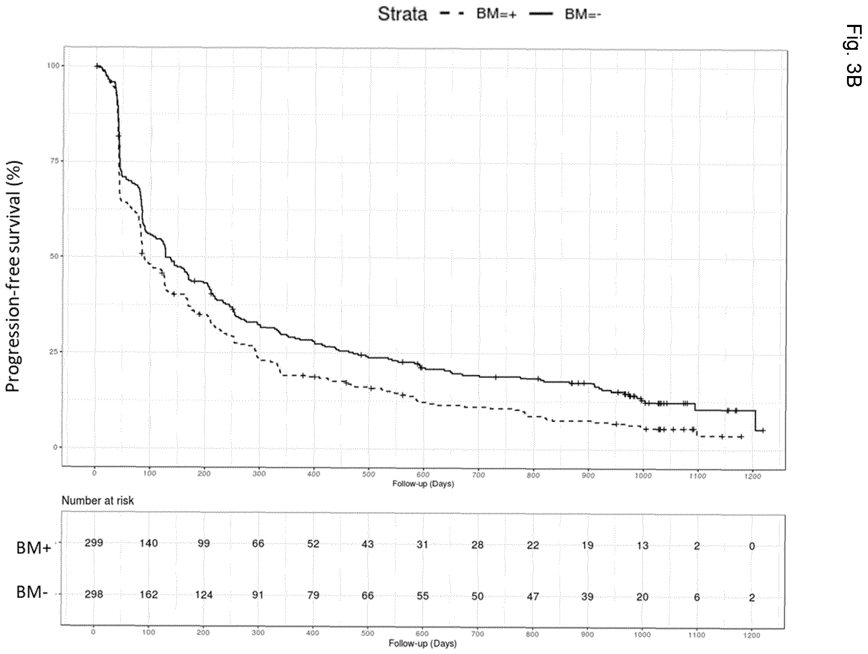

As several genes linked to stem cell maintenance-related cancer cells were associated with the survival advantages, we investigated the impact of multiple genes involved in stem cell maintenance-related cancer cells by defining a cumulative cancer stem cell gene score reflecting the cumulative expression of these marker genes. Each gene's expression is first standardized by a z-score: $z = \frac{x - μ}{σ} ,$ where µand µ are estimated in the entire cohort or in the selected subgroups. After the standardization step, these standardized z-score values are averaged across genes within each patient. The BIRCH cohort was corrected for the sex of patient, age as well as smoking status. Based on such a analysis, we observed that patients treated with Atezolizumab and a lower cancers stem cell score showed superior survival advantage, having a medium overall survival of ~583 days, whereas the high expression group had a medium overall survival of ~437 days resulting in HR=0.77 and p=0.01 (**Figure 3A**). In addition, we also identified an advantage in progression-free survival. Patients treated with Atezolizumab and a lower cancers stem cell score showed a median progression-free survival of ~127 days and the high group of ~89 days resulting in an HR of 0.74 and p=0.0007 (**Figure 3B**).

### Example 3

### Stemness-related gene transcripts predict clinical benefit in patients with With Locally Advanced or Metastatic Urothelial Bladder Cancer treated with atezolizumab.

We hypothesized that patients with a low abundance of stem cell maintenance-related cancer cells may respond to PD-L1 blockade leading to beneficial effect in patients who received the treatment. We analyzed **750** patients with locally advanced or metastatic urothelial bladder cancer who received Atezolizumab (n=369), Paclitaxel (n=123), Vinflunine (n=194) or Docetaxel (n=41) in a Phase III clinical trial (NCT02302807, IMvigor 211) (https://clinicaltrials.gov/ct2/show/NCT02302807). This study was sponsored by Genentech Inc., a member of the Roche Group, which provided the study drug. The protocol and its amendments were approved by the relevant institutional review boards or ethics committees, and all participants provided written informed consent. This study was conducted in accordance with the Declaration of Helsinki and International Conference on Harmonization Guidelines for Good Clinical Practice.

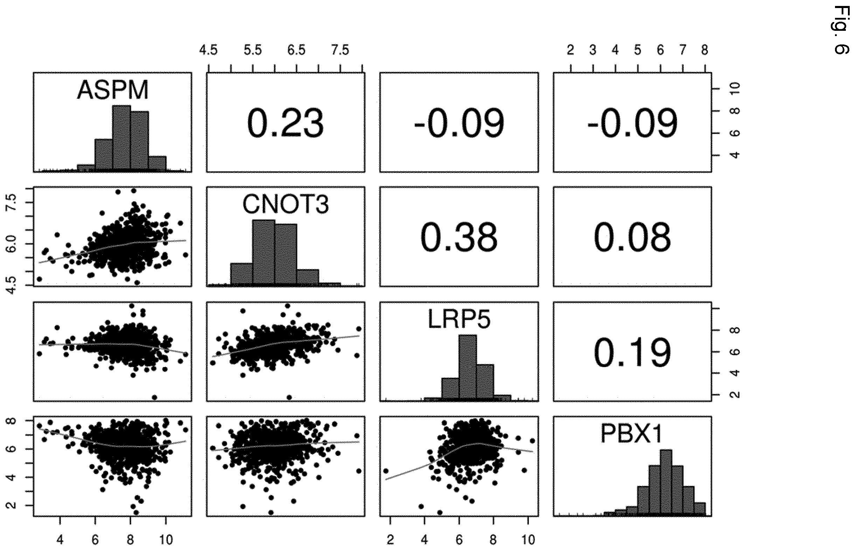

Tumor specimens at baseline were archived and taken for gene expression profiling performed by RNA Sequencing (RNA-Seq). In total, 144 human stem cell related genes derived from Gene Ontology were selected and further processed resulting in a selected a list of 4 genes consisting of ASPM, CNOT3, LRP5, and PBX1, which are associated with abundance of stem cell maintenance-related cancer cells. The log2 CPM expression values of each selected gene across the whole cohort were divided at medium expression level for the higher expression ones (+) and lower/no expression ones (-). Using in-house R scripts, the two defined subgroups were plotted against the Kaplan-Meier survival curves. **Figure 6** shows the correlation of single gene expression pattern with the patient survival. **Table 3** summarizes the median survival as well as HR per single gene used for the stem cell maintenance-related cancer cells signature.

**Table 3: single gene association with the survival advantages by a PD-1 axis inhibitor Atezolizumab**

| **Gene** | **Treatment** | **Signature** | **Median survival (days)** | **HR (low vs high)** | **HR (Atezo vs SoC)** |
|---|---|---|---|---|---|
| ASPM | SoC* | Stem cell score high | 227 | | |
| | | Stem cell score low | 249 | | HR: 0.93 |
| | Atezolizumab | Stem cell score low | 257 | HR: 1.05 | pval: 0.57 |
| | | Stem cell score high | 271 | pval: 0.72 | |
| CNOT3 | SoC* | Stem cell score high | 217 | | |
| | | Stem cell score low | 262 | | HR: 0.83 |
| | Atezolizumab | Stem cell score low | 297 | HR: 0.85 | pval: 0.13 |
| | | Stem cell score high | 242 | pval: 0.19 | |
| LRP5 | SoC* | Stem cell score high | 224 | | |
| | | Stem cell score low | 249 | | HR: 0.74 |
| | Atezolizumab | Stem cell score low | 298 | HR: 0.76 | pval: 0.02 |
| | | Stem cell score high | 241 | pval: 0.03 | |
| PBX1 | SoC* | Stem cell score high | 261 | | |
| | | Stem cell score low | 220 | | HR: 0.67 |
| | Atezolizumab | Stem cell score low | 279 | HR: 0.74 | pval: 0.001 |
| | | Stem cell score high | 247 | pval: 0.02 | |

| | | | | | |
|---|---|---|---|---|---|
| SoC* is standard of care treatment with vinflunine or paclitaxel or docetaxel. | | | | | |

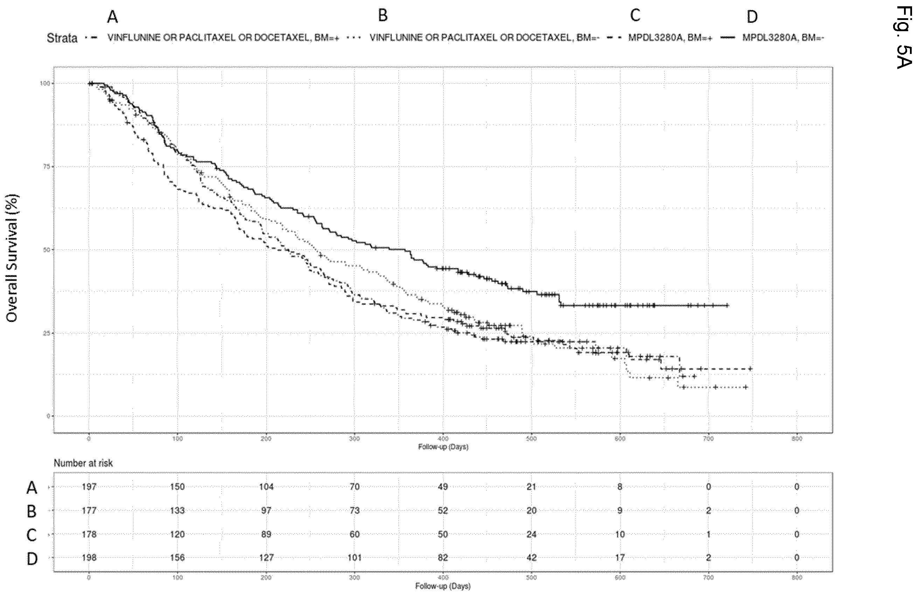

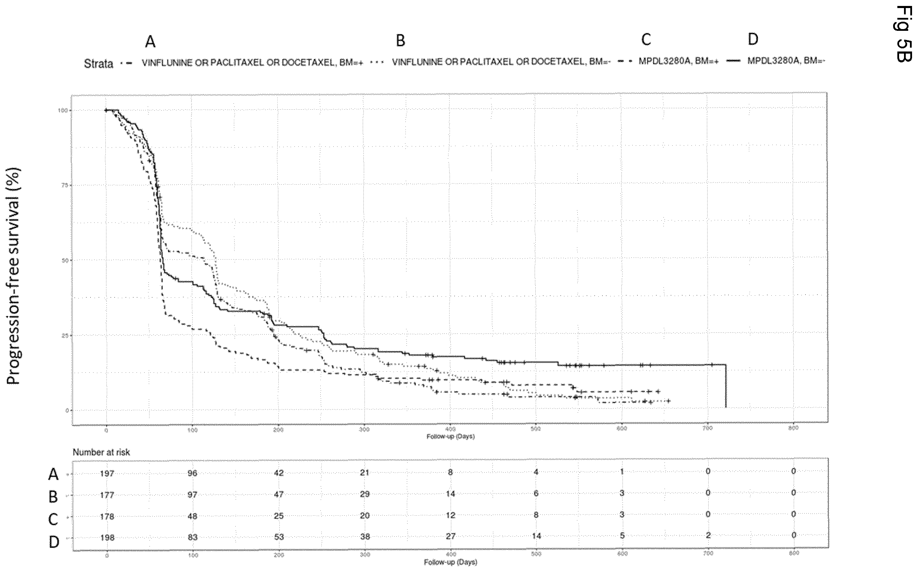

As several genes linked to stem cell maintenance-related cancer cells were associated with the survival advantages, we investigated the impact of multiple genes involved in stem cell maintenance-related cancer cells by defining a cumulative cancer stem cell gene score reflecting the cumulative expression of these marker genes. Each gene's expression is first standardized by a z-score: $z = \frac{x - μ}{σ} ,$ where µand µ are estimated in the entire cohort or in the selected subgroups. After the standardization step, these standardized z-score values are averaged across genes within each patient. The IMvigor211 cohort was corrected for the sex of patient, age as well as smoking status. Based on such a analysis, we observed that patients treated with Atezolizumab and a lower cancers stem cell score showed superior overall survival advantage, having a medium overall survival of ~357 days, whereas the high expression group had a medium overall survival of ~211 days (HR=0.64, and p=0.0004) (**Figure 5A**). Comparing, patients having a low cancers stem cell score, the standard of care group (combining VINFLUNINE, PACLITAXEL or DOCETAXEL) subgroup shows a median overall survival of ~256 days (high subgroup ~225 days). Therefore, the stem cell score low groups shows a predicitve effect for Atezolizumab (HR=0.70, and p=0.004). In addition, we also identified an adavantage in progression-free survival. Patients treated with Atezolizumab and a lower cancers stem cell score showed a median progression-free survival of ~66 days and the high group of ~63 days resulting in an HR of 0.69 and p=0.001 (**Figure 5B**).

## Claims

1. An *in vitro* method of identifying a patient having cancer who is responsive to a therapy comprising an effective amount of an anti-PD-L1 antibody, the method comprising determining the abundance of stem cell maintenance-related genes in a tumor tissue sample obtained from a patient having cancer, wherein the abundance of stem cell maintenance-related genes is **characterized by** detecting the expression level of all genes selected from a group consisting of ASPM, CNOT3, LRP5 and PBX1 and wherein the method further comprises a step of comparing the expression level of the genes to a reference level, whereby an increased expression level is indicative of response to a therapy comprising an effective amount of an anti-PD-L1 antibody.

2. The method of claim 1, wherein the expression level is detected in the sample by protein expression.

3. The method of claim 1, wherein the expression level is detected in the sample by mRNA expression.

4. The method of any one of claims 1 to 3, wherein the expression level is detected using a method selected from the group consisting of FACS, Western blot, ELISA, immunoprecipitation, immunohistochemistry, immunofluorescence, radioimmunoassay, immunodetection methods, mass spectrometery, HPLC, qPCR, RT-qPCR, multiplex qPCR or RT-qPCR, RNA-seq, microarray analysis, nanostring, SAGE, MassARRAY technique, and FISH, and combinations thereof.

5. The method of any one of claims 1 to 4, wherein the cancer is locally advanced or metastatic non-small cell lung cancer or urothelial bladder cancer.

6. The method of any one of claims 1 to 5, wherein the therapy comprises an effective amount of an anti-PD-L1 antibody as monotherapy.

7. The method of any one of claims 1 to 6, wherein the therapy comprises an effective amount of an anti-PD-L1 antibody and an effective amount of a second agent selected from the group consisting of a cytotoxic agent, a chemotherapeutic agent, a growth inhibitory agent, a radiation therapy agent, and anti-angiogenic agent, and combinations thereof.

8. The method of claim 1, wherein the anti-PD-L1 antibody is selected from the group consisting of atezolizumab, avelumab, durvalumab and MDX-1105.

9. The method of any one of claims 1 to 8, wherein the tumor tissue sample is a sample obtained from the patient prior to the therapy with an anti-PD-L1 antibody.

## Patentansprüche

1. *In*-*vitro*-Verfahren zum Identifizieren eines Krebspatienten, der auf eine Therapie anspricht, die eine wirksame Menge eines Anti-PD-L1-Antikörpers umfasst, wobei das Verfahren die Bestimmung der Expressionsstärke von Genen umfasst, die mit der Erhaltung von Stammzellen in Zusammenhang stehen, in einer Tumorgewebeprobe, die von einem Krebspatienten entnommen wurde, wobei die Expressionsstärke der Gene, die mit der Erhaltung von Stammzellen in Zusammenhang stehen, durch den Nachweis des Expressionsniveaus aller Gene charakterisiert wird, ausgewählt aus einer Gruppe, bestehend aus ASPM, CNOT3, LRP5 und PBX1, und wobei das Verfahren ferner einen Schritt umfasst, bei dem das Expressionsniveau der Gene mit einem Referenzniveau verglichen wird, wobei ein erhöhtes Expressionsniveau auf ein Ansprechen auf eine Therapie hinweist, die eine wirksame Menge eines Anti-PD-L1-Antikörpers umfasst.

2. Verfahren nach Anspruch 1, wobei das Expressionsniveau in der Probe durch Proteinexpression nachgewiesen wird.

3. Verfahren nach Anspruch 1, wobei das Expressionsniveau in der Probe durch mRNA-Expression nachgewiesen wird.

4. Verfahren nach einem der Ansprüche 1 bis 3, wobei das Expressionsniveau unter Verwendung eines Verfahrens nachgewiesen wird, ausgewählt aus der Gruppe, bestehend aus FACS, Western Blot, ELISA, Immunpräzipitation, Immunhistochemie, Immunfluoreszenz, Radioimmunoassay, Immunnachweisverfahren, Massenspektrometrie, HPLC, qPCR, RT-qPCR, Multiplex-qPCR oder RT-qPCR, RNA-seq, Mikroarray-Analyse, Nanostring, SAGE, MassARRAY-Technik und FISH und Kombinationen davon.

5. Verfahren nach einem der Ansprüche 1 bis 4, wobei der Krebs lokal fortgeschrittener oder metastasierender nicht-kleinzelliger Lungenkrebs oder Urothelblasenkrebs ist.

6. Verfahren nach einem der Ansprüche 1 bis 5, wobei die Therapie eine wirksame Menge eines Anti-PD-L1-Antikörpers als Monotherapie umfasst.

7. Verfahren nach einem der Ansprüche 1 bis 6, wobei die Therapie eine wirksame Menge eines Anti-PD-L1-Antikörpers und eine wirksame Menge eines zweiten Mittels umfasst, ausgewählt aus der Gruppe, bestehend aus einem zytotoxischen Mittel, einem chemotherapeutischen Mittel, einem wachstumshemmenden Mittel, einem Strahlentherapiemittel und einem antiangiogenen Mittel und Kombinationen davon.

8. Verfahren nach Anspruch 1, wobei der Anti-PD-L1-Antikörper aus der Gruppe ausgewählt ist, bestehend aus Atezolizumab, Avelumab, Durvalumab und MDX-1105.

9. Verfahren nach einem der Ansprüche 1 bis 8, wobei die Tumorgewebeprobe eine Probe ist, die von dem Patienten vor der Therapie mit einem Anti-PD-L1-Antikörper entnommen wurde.

## Revendications

1. Méthode *in vitro* d'identification d'un patient atteint d'un cancer qui est sensible à une thérapie comprenant une quantité efficace d'un anticorps anti-PD-L1, la méthode comprenant la détermination de l'abondance de gènes liés au maintien des cellules souches dans un échantillon de tissu tumoral obtenu auprès d'un patient atteint d'un cancer, dans laquelle l'abondance de gènes liés au maintien des cellules souches est **caractérisée par** la détection du niveau d'expression de tous les gènes choisis dans le groupe constitué par ASPM, CNOT3, LRP5 et PBX1 et dans laquelle la méthode comprend en outre une étape de comparaison du niveau d'expression des gènes à un niveau de référence, moyennant quoi un niveau d'expression accru indique une réponse à une thérapie comprenant une quantité efficace d'un anticorps anti-PD-L1.

2. Méthode selon la revendication 1, dans laquelle le niveau d'expression est détecté dans l'échantillon par l'expression de protéines.

3. Méthode selon la revendication 1, dans laquelle le niveau d'expression est détecté dans l'échantillon par l'expression d'ARNm.

4. Méthode selon l'une quelconque des revendications 1 à 3, dans laquelle le niveau d'expression est détecté en utilisant une méthode choisie dans le groupe constitué par une FACS, un transfert de Western, un ELISA, une immunoprécipitation, une immunohistochimie, une immunofluorescence, un dosage radioimmunologique, des méthodes d'immunodétection, une spectrométrie de masse, une CLHP, une qPCR, une RT-qPCR, une qPCR ou une RT-qPCR multiplex, un RNA-seq, une analyse par microréseau, une analyse nanostring, une analyse SAGE, une technique MassARRAY et une analyse FISH, et des combinaisons de ceux-ci.

5. Méthode selon l'une quelconque des revendications 1 à 4, dans laquelle le cancer est un cancer du poumon non à petites cellules localement avancé ou métastatique ou un cancer urothélial de la vessie.

6. Méthode selon l'une quelconque des revendications 1 à 5, dans laquelle la thérapie comprend une quantité efficace d'un anticorps anti-PD-L1 en monothérapie.

7. Méthode selon l'une quelconque des revendications 1 à 6, dans laquelle la thérapie comprend une quantité efficace d'un anticorps anti-PD-L1 et une quantité efficace d'un second agent choisi dans le groupe constitué par un agent cytotoxique, un agent chimiothérapeutique, un agent inhibiteur de croissance, un agent de radiothérapie et un agent anti-angiogénique, et des combinaisons de ceux-ci.

8. Méthode selon la revendication 1, dans laquelle l'anticorps anti-PD-L1 est choisi dans le groupe constitué par l'atézolizumab, l'avélumab, le durvalumab et MDX-1105.

9. Méthode selon l'une quelconque des revendications 1 à 8, dans laquelle l'échantillon de tissu tumoral est un échantillon obtenu auprès du patient avant la thérapie avec un anticorps anti-PD-L1.
